# FASCICULE DE BREVET EUROPEEN

(11) **EP 3 205 629 B1**
(45) Date de publication et mention de la délivrance du brevet: **23.01.2019**
(21) Numéro de dépôt: 17155588.1
(22) Date de dépôt: 10.02.2017
(51) Int. Cl.: C02F 11/04, G01N 21/3563, G01N 33/00, G01N 21/359, C12M 1/107, C12M 1/36

(54) **PROCÉDÉ D'ESTIMATION DE LA BIODÉGRADATION D'UN SUBSTRAT DANS UN MÉTHANISEUR**
PROZESS ZUR ABSCHÄTZUNG DER BIODEGRADATION EINES SUBSTRATS IN EINEM FERMENTER
PROCEDURE FOR ESTIMATING THE BIODEGRADATION OF A SUBSTRATE IN A DIGESTER

(30) Priorité: 10.02.2016 FR 1651050
(43) Date de publication de la demande: 16.08.2017
(73) Titulaire: BIOENTECH, 11100 Narbonne (FR); Institut National de la Recherche Agronomique (INRA), 75338 Paris Cedex 07 (FR)
(72) Inventeur: CHARNIER, Cyrille, 11590 CUXAC D'AUDE (FR); LATRILLE, Eric, 11110 SALLES D'AUDE (FR); JIMENEZ, Julie, 11100 NARBONNE (FR); MIROUX, Jérémie, 69780 SAINT PIERRE DE CHANDIEU (FR); STEYER, Jean-Philippe, 11200 NEVIAN (FR); TORRIJOS, Michel, 11100 NARBONNE (FR); SOUSBIE, Philippe, 11120 MOUSSAN (FR); LEMOINE, Margaux, 11110 VINASSAN (FR)
(74) Mandataire: Agasse, Stéphane

(56) Documents cités:
- GALÍ A ET AL: "Modified version of ADM1 model for agro-waste application", BIORESOURCE TECHNOLOGY, vol. 100, no. 11, 2009, pages 2783-2790, XP055385606, GB ISSN: 0960-8524, DOI: 10.1016/j.biortech.2008.12.052
- GRIEDER C ET AL: "Determination of methane fermentation yield and its kinetics by near infrared spectroscopy and chemical composition in maize", JOURNAL OF NEAR INFRARED SPECTROSCOPY, vol. 19, no. 6, 9 janvier 2012 (2012-01-09), pages 463-477, XP055325353, GB ISSN: 0967-0335, DOI: 10.1255/jnirs.959

## Description

La présente invention concerne l'estimation des performances de la biodégradation d'un substrat par digestion anaérobie dans un méthaniseur, cette estimation étant notamment réalisée avec un modèle de prédiction des caractéristiques du substrat, et ce afin que l'opérateur puisse disposer d'informations sur ce qui va se produire dans le méthaniseur préalablement à l'incorporation du substrat. Les informations ainsi recueillies avant la mise en oeuvre de la biodégradation du substrat dans le méthaniseur sont établies en fonction des caractéristiques du substrat.

La digestion anaérobie présente un fort intérêt environnemental du fait de son efficacité dans la valorisation énergétique de différents types de substrats qui comprennent essentiellement des déchets de nature très variée. Elle est ainsi destinée, dans le futur, à jouer un rôle majeur dans la production d'énergies renouvelables.

En effet, au cours de la digestion anaérobie dans un méthaniseur, une partie de la matière organique du substrat est convertie en un biogaz riche en énergie qui comprend essentiellement du méthane (ci-après abrégé « CH₄ ») et du dioxyde de carbone (ci-après abrégé « CO₂ »).

Le méthane étant le gaz majoritairement produit au cours de la digestion anaérobie, dans la présente description de l'invention, les termes « méthaniseur » et « digesteur » sont employés de manière parfaitement équivalente.

Le substrat biodégradé au cours de la digestion anaérobie peut être de nature très variée. Il s'agit par exemple :
- des eaux usées industrielles, ainsi que des eaux usées urbaines qui contiennent des particules, notamment des particules solides, contenant un substrat à biodégrader ;
- des boues de station d'épuration qui contiennent également des particules, notamment des particules solides, contenant un substrat à biodégrader;
- des micro-algues ;
- des déchets municipaux solides qui se décomposent en différentes catégories :
   ∘ les matières putrescibles telles que les déchets alimentaires et les herbes ;
   ∘ les papiers, les cartons ;
- des déchets industriels, en particulier les matières grasses, les graisses et les huiles connus sous l'acronyme de « FOG » pour l'abréviation anglophone de « Fats, Oils, Greases » ;
- des déchets de l'agriculture tels que par exemple l'ensilage, le fumier, les résidus de céréales et le lactosérum.

La digestion anaérobie consiste en une multitude de réactions qui sont pour la plupart de nature biochimique.

Les étapes principales de la digestion anaérobie sont les suivantes :
- la 1^{ière} étape consiste en une dégradation de la matière organique particulaire en des glucides, lipides et protéines ;
- la 2^{ième} étape consiste en une hydrolyse enzymatique de ces composés en des glucides à chaînes plus courtes, des acides gras à longues chaînes et des acides aminés. Les enzymes hydrolytiques sont secrétées par des microorganismes présents dans le liquide du substrat ou attachés aux particules que comprend le substrat ;
- la 3^{ième} étape consiste en une acidogénèse réalisée par les microorganismes présents dans le digesteur qui convertissent les composés solubles obtenus à l'issue de l'hydrolyse en des alcools et/ou des acides organiques tels que l'acétate, le propionate, le butyrate, le valérate ;
- la 4^{ième} étape consiste en une acétogénèse réalisée grâce aux microorganismes présents dans le digesteur qui transforment les composés obtenus à l'issue de l'acidogénèse en des acétates, dioxyde de carbone et dihydrogène ;
- la 5^{ième} étape consiste en une méthanogénèse réalisée grâce aux microorganismes présents dans le digesteur qui convertissent les composés obtenus à l'issue de l'acétogénèse (acétate, dioxyde de carbone et dihydrogène) en méthane.

La composition du biogaz obtenu à l'issue de la digestion anaérobie dans un méthaniseur dépend de différents facteurs que sont notamment :
- l'état d'oxydation du carbone dans le substrat ;
- le temps de séjour dans le digesteur ;
- les paramètres de réglages du digesteur. Par exemple, un digesteur continu favorise une faible teneur en dioxyde de carbone du fait que le liquide dans le méthaniseur va se charger en dioxyde de carbone dissous. De plus, la température du digesteur peut influencer les cinétiques et la solubilité des gaz produits au cours de la digestion anaérobie ;
- la quantité d'hydrogène dissous : de fortes concentrations en hydrogène dissous vont inhiber les étapes d'acidogénèse alors que l'hydrogène va être un composé nécessaire pour la méthanogénèse.

C'est pourquoi, en vue d'en améliorer ses performances, la digestion anaérobie fait l'objet d'intenses recherches sur différents sujets que sont notamment :
- les caractéristiques du digesteur ;
- la caractérisation des substrats ;
- le prétraitement du substrat pour améliorer la biodégradabilité du substrat et le rendement de biogaz ;
- les microorganismes du digesteur.

En parallèle à cela, des recherches ont également été entreprises pour comprendre plus précisément les différents processus de la digestion anaérobie qui se produisent dans un digesteur et qui sont bien plus complexes que les étapes principales détaillées ci-dessus, et ce en vue d'optimiser la digestion anaérobie, à savoir d'augmenter le rendement de biogaz produit. Cette meilleure compréhension de la digestion anaérobie a consisté en le développement de modèles mathématiques de digestion anaérobie qui permettent de simuler tout procédé de digestion anaérobie, autrement dit de prédire ce qui va se produire au sein du digesteur, afin d'en optimiser ses performances.

Parmi ces modèles mathématiques de digestion anaérobie, il existe le modèle « ADM1 » (à savoir l'acronyme anglophone pour « Anaerobic Digestion Model n°1 ») qui décrit les réactions se produisant au cours de la digestion anaérobie et en supposant que le mélange au sein du méthaniseur est parfait. Le modèle ADM1 a fait notamment l'objet de cette publication de 2002 : Batstone, D.J., Keller, J., Angelidaki, I.., Kalyuzhnyi, S., Pavlostathis, S.G., Rozzi, A., Sanders, W., Siegrist, H. and Vavilin, V. (IWA Task Group on Modelling of Anaerobic Digestion Processes) (2002). Anaerobic Digestion Model No. 1 (ADM1). IWA Publishing, London.

Le modèle ADM1 tient compte aussi bien des processus biochimiques que physicochimiques qui interviennent au cours de la digestion anaérobie.

Le modèle mathématique ADM1 a plusieurs variantes de modèles qui font toujours l'objet de recherches en vue de perfectionner ce modèle quant à la justesse de l'estimation des caractéristiques de la digestion anaérobie qu'il peut fournir.

La publication de Galí, A. et al., "Modified version of ADM1 model for agro-waste application", Bioresour. Technol. 100 (2009), doi: 10.1016/j.biortech.2008.12.052, décrit un modèle ADM1 modifié pour simuler la digestion anaérobie dont les substrats sont des déchets de l'agro-alimentaire. Les caractéristiques du substrat et les caractéristiques de désintégration ont été déterminées à partir d'analyses du substrat. Les constantes de désintégration sont obtenues à partir de la quantité cumulée de production du méthane. Après avoir déterminé les caractéristiques du substrat et les constantes de désintégration, un modèle ADM1 a été établi qui a ensuite été validé avec différentes conditions de fonctionnement du digesteur.

Ainsi, un des grands challenges de la digestion anaérobie consiste en la prédiction des performances du procédé de digestion anaérobie avant l'ajout du substrat dans le digesteur à l'aide de modèles mathématiques tels que par exemple le modèle ADM1, et ce de préférence dans un lapse de temps le plus court possible et en adéquation avec les contraintes opérationnelles des digesteurs.

De plus, pour les substrats solides, les étapes limitantes de la digestion anaérobie sont la dégradation et l'hydrolyse. Ces deux étapes sont expérimentalement difficiles à séparer. La séparation de ces étapes de dégradation et d'hydrolyse est le plus souvent utilisée à des fins de modélisation. Ces étapes sont spécifiques au substrat et nécessitent donc pour chaque nouveau substrat de modifier les paramètres de caractérisation du substrat qui sont implémentés dans un modèle mathématique de digestion anaérobie si l'on veut obtenir la prédiction la plus juste possible du fonctionnement du méthaniseur, autrement dit l'estimation la plus juste des caractéristiques de la biodégradation d'un substrat par digestion anaérobie dans ledit méthaniseur.

Ces caractéristiques d'un substrat sur la base desquelles sont implémentés les modèles mathématiques de digestion anaérobie tels que le modèle ADM1 afin de décrire avec la plus grande justesse possible le procédé de digestion anaérobie peuvent être :
1) la teneur en lipides ;
2) la teneur en sucres ;
3) la teneur en protéines ;
4) la demande chimique en oxygène, ci-après abrégée avec l'acronyme « DCO » ;
5) la fraction de matière organique dégradable ;
6) le potentiel biochimique de méthane, ci-après abrégé « BMP », à savoir l'acronyme anglophone de « Biochemical Methane Potentiel » ;
7) la cinétique de dégradation de la matière organique du substrat, ci-après abrégée « la cinétique de dégradation du substrat » ;
8) l'accessibilité du substrat ;
9) la biodégradabilité du substrat.

Une fois le modèle mathématique de digestion anaérobie implémenté, il est possible de simuler tout scénario d'alimentation en substrat souhaité afin de choisir la recette d'alimentation optimale pour le digesteur. Il est fondamental de caractériser le substrat avec la plus grande justesse possible afin de pouvoir implémenter au mieux les modèles mathématiques de digestion anaérobie et d'obtenir une prédiction des performances du méthaniseur la plus juste possible.

Or, comme cela a été rappelé ci-dessus, les substrats utilisés dans des digesteurs sont souvent de nature très variée et complexe. Les déchets municipaux solides sont un exemple qui illustre parfaitement cette complexité. Non seulement leur composition est complexe, mais elle peut en outre évoluer au cours du temps selon leurs différents approvisionnements. Cette variabilité du substrat influe grandement sur les performances d'un digesteur et peut donc avoir un impact non négligeable sur sa rentabilité.

Il est ainsi essentiel de disposer de moyens prédictifs pour anticiper le comportement du digesteur face à une variation de la composition de la recette d'alimentation du digesteur.

C'est pourquoi, on cherche à caractériser les substrats avant de les introduire dans un digesteur afin d'améliorer la production de méthane en sélectionnant les substrats les plus productifs et en créant des mélanges de substrats adéquats. En effet, la fourniture à l'opérateur d'une estimation du gain obtenu selon le choix d'un substrat est un excellent atout pour l'élaboration de la recette d'alimentation pour optimiser le fonctionnement et la rentabilité du digesteur.

Aujourd'hui, des méthodes de laboratoire existent pour estimer les caractéristiques d'un substrat sur la base desquels peuvent être implémentés les modèles mathématiques de digestion anaérobie.

A cet égard, on peut citer les méthodes de laboratoire suivantes :
La teneur en lipides d'un substrat peut être déterminée par une extraction par solvant. La quantité de lipides extraits se mesure en quantifiant la masse de lipides récupérés dans le solvant après évaporation de ce dernier. Par exemple, le solvant peut être de l'heptane qui est évaporé sous flux d'azote. Le résidu est ensuite séché et la masse de celui-ci est pesée. Ainsi, on obtient une teneur en lipides du substrat qui est exprimée en gramme sec par gramme de matière sèche.

La teneur en glucides d'un substrat (autrement dit sa teneur en sucres) peut être déterminée avec une méthode colorimétrique. Par exemple, cette méthode peut consister à estimer la concentration en sucre d'un échantillon en équivalent glucose (ci-après abrégé « Glu »). Cette méthode est décrite sur une plage de linéarité de 0 à 100 mg Glu/L. L'étalonnage standard est réalisé avec du glucose. La méthode se base sur la formation de produits d'hydrolyse acide (acide sulfurique 95% g/g). Ces produits sont ensuite condensés avec du phénol en chromophores orange qui présente une absorption à 490 nm. L'intensité de coloration est proportionnelle à la concentration équivalente de glucose.

La teneur en protéines d'un substrat peut être déterminée en mesurant l'azote total selon la méthode de Kjeldhal avec hydrolyse acide. Le principe est une décomposition totale de la matière par hydrolyse acide. L'azote minéral qui en résulte est ensuite titré. Un ratio de conversion permet d'obtenir la teneur en protéines à partir de la teneur en azote ainsi déterminée expérimentalement.

La DCO est la quantité de dioxygène nécessaire pour oxyder de la matière organique. Elle est un indicateur de la quantité d'énergie dans le substrat. La détermination de la DCO peut être effectuée sur des phases solubles et totales en utilisant un procédé de kits commerciaux correspondant à une mesure de densité optique proportionnelle à la quantité d'oxygène nécessaire à l'oxydation de la matière organique introduite dans le kit suite à la loi de Beer-Lambert. La mesure de la DCO représente l'ensemble des matières organiques oxydables. La réaction d'oxydation est effectuée par le dichromate de potassium dans des conditions acides, à 150°C pendant 2 heures. L'oxydation de la matière produit des ions Cr³⁺ de coloration verte. La mesure des concentrations est réalisée avec un spectrophotomètre.

La biodégradabilité est définie comme le rapport entre la quantité maximale de méthane potentiellement produite par la digestion d'un substrat (à savoir le BMP) et la production réelle de méthane, soit BMP/(DCO*350), le BMP étant exprimé en mL CH4/g de matière sèche et la DCO est exprimée en g de dioxygène par g de matière sèche.

Le BMP correspond à la quantité maximale de méthane potentiellement produite par la digestion anaérobie d'un substrat dans des conditions optimales. La détermination du BMP est réalisée à partir d'une expérimentation biologique de dégradation du substrat en condition anaérobie consistant en le relevé de la production de méthane. Cette expérimentation dure généralement environ un mois.

Le BMP d'un substrat peut être déterminé selon une analyse de laboratoire telle que décrite par exemple dans la publication de Garcia-Gen, S. et al, Kinetic modelling of anaerobic hydrolysis of solid wastes, including disintegradation processes. Waste Management. (2014) doi : 10.1016/j.wasman. 2014.10.012.

A des fins de modélisation, le paramètre de cinétique de dégradation est estimé en minimisant l'erreur sur la production de méthane entre une expérience de digestion anaérobie et un modèle, par exemple un modèle ADM1. Cette expérience peut être réalisée dans un réacteur de 6 litres de volume effectif. Ce réacteur est inoculé avec des boues issues d'un digesteur industriel. Il est alimenté pendant 6 à 8 semaines à raison de 1g de matière volatile par semaine. Le réacteur est suivi par sa production en biogaz. La cinétique de dégradation est déterminée sur le dernier batch considéré comme le plus représentatif car la biomasse est adaptée au substrat. Sur celui-ci est aussi estimé le BMP.

L'accessibilité du substrat peut être analysée par extraction chimique suivant un protocole de fractionnement parfaitement connu de l'homme du métier. Un exemple de mise en oeuvre d'un tel protocole est détaillé dans la publication de Jimenez, J. et al, A new organic matter fractionation methodology for organic wastes: bioaccessibility and complexity characterization for treatment optimization, Bioresour. Technol. (2015), doi :10.1016/j.biortech. 2015.07.037. Des solvants de plus en plus forts sont utilisés sur la matière organique, à titre d'exemple :
- les matières extraites lors de l'extraction avec un solvant contenant un mélange de soude à une concentration de 0,01 mol/L et de chlorure de sodium de 0,4 g/L sont dites « facilement accessibles » (ci-après abrégé « REOM »),
- les matières extraites lors de l'extraction avec un solvant contenant un mélange de soude à une concentration de 0,1 mol/L sont dites « moyennement accessibles » (ci-après abrégé « MEOM »),
- les matières extraites lors de l'extraction avec un solvant contenant de l'acide sulfurique concentré à 72% en volume sont dites « difficilement accessibles (ci-après abrégé « SEOM »),
- les matières restantes après ces extraction sont dites « non extractibles » (ci-après abrégé « NEOM »).

Ainsi, à l'issue de ce protocole de fractionnement, on dispose des trois factions contenant donc les matières extraites suivantes : REOM, MEOM, SEOM et des trois culots suivants : 1^{ier} culot de MEOM, SEOM et NEOM ; 2^{ième} culot de SEOM et NEOM et 3^{ième} culot de NEOM.

Toutefois, ces méthodes de laboratoire présentent les inconvénients d'êtres onéreuses et surtout très longues. Leur durée est parfois comprise entre 1 et 2 mois. Elles sont, par conséquent, peu adaptées à la gestion de l'approvisionnement en substrats des méthaniseurs. En effet, dans le domaine de la digestion anaérobie, on minimise les durées de stockage du substrat, car tout l'intérêt de ce procédé réside dans l'élimination de déchets en produisant les meilleurs rendements de biogaz. Le stockage du substrat durant le temps de l'analyse laboratoire d'un échantillon de ce substrat limiterait alors l'intérêt de la digestion anaérobie.

Ainsi, la détermination des caractéristiques d'un substrat avec les méthodes de laboratoire n'est pas appropriée pour le domaine technique des méthaniseurs.

La publication de Lesteur, M. et al. First step towards a fast analytical method for the détermination of biochemical methane potential of solids wastes by near infrared spectroscopy. Bioresour. Technol. (2010), doi :10.1016/j.biortech. 2010.10.44 décrit une méthode qui utilise la spectroscopie proche infra-rouge (ci-après abrégé « NIR » qui est l'acronyme anglophone de « near infrared ») pour prédire le BMP d'un substrat constitué de déchets solides municipaux. Cette méthode permettrait de s'affranchir de la méthode de laboratoire utilisée pour la détermination du BMP d'un substrat.

La publication de Grieder, C. et al., "Détermination of methane fermentation yield and its kinetics by near infrared spectroscopy and chemical composition in maize", J. Near Infrared Spectroscopy 19 (2011), doi:10.1255/jnirs.959, décrit une méthode pour prédire le rendement de fermentation du méthane ("Methane fermentation yield", MFY) par des analyses de spectroscopie proche infra-rouge (NIR) et vérifie la fiabilité des prédictions de paramètres liés à la cinétique du MFY. Plus précisément, il est déterminé à quels stades de la fermentation les prédictions du MFY obtenues avec les analyses NIR sont les meilleures. Les meilleures prédictions sont obtenues en début de fermentation.

Cependant, la seule prédiction du BMP d'un substrat destiné à être biodégradé dans un méthaniseur ne permet pas de caractériser de manière satisfaisante et utile pour l'opérateur ce substrat avant sa biodégradation par digestion anaérobie. En effet, le BMP ne rend pas compte des interactions entre les substrats dans un processus de co-digestion. De plus, un méthaniseur est lié à des conditions opératoires telles que le temps de séjour (à savoir le temps moyen qu'une particule passe dans le digesteur). Par conséquent, la simple connaissance du BMP d'un substrat avant son incorporation dans un méthaniseur ne permet pas de prédire la production de méthane qui aura lieu dans ledit méthaniseur, car ce paramètre n'est relié à aucune donnée cinétique. Par exemple, si le temps nécessaire pour atteindre la valeur du BMP est de 50 jours alors que le temps de séjour est de 25 jours, le BMP ne pourra jamais être atteint dans le digesteur sans que l'opérateur ne puisse diagnostiquer l'origine du problème qui pourrait être due à l'agitation du méthaniseur, à la présence d'inhibiteurs ou encore à un temps de digestion trop court.

La présente invention se propose de surmonter les inconvénients tels que détaillés ci-dessus quant aux difficultés liées à l'estimation des performances de la biodégradation d'un substrat par digestion anaérobie dans un méthaniseur du fait notamment des caractéristiques variées et complexes dudit substrat.

La présente invention a ainsi pour objet un procédé d'estimation d'au moins une des caractéristiques de la biodégradation d'un substrat par digestion anaérobie dans un méthaniseur qui comprend au moins les étapes suivantes :
a) on réceptionne des données spectrales dans le NIR d'un échantillon dudit substrat en provenance d'un système d'analyse de spectroscopie ou on acquiert des données spectrales dans le NIR d'un échantillon dudit substrat ;
b) à partir desdites données spectrales dans le NIR de l'échantillon du substrat, on prédit des caractéristiques dudit substrat avec au moins un modèle de prédiction des caractéristiques d'un substrat destiné à être incorporé dans un méthaniseur pour être biodégradé par digestion anaérobie, lesdites caractéristiques prédites du substrat comprennent au moins :
   - le BMP dudit substrat,
   - le temps nécessaire pour la production de méthane exprimé en pourcentage de BMP du substrat ;
c) à partir des caractéristiques prédites consistant en le BMP du substrat et en le temps nécessaire pour la production de méthane exprimé en pourcentage de BMP du substrat, on établit une courbe de la quantité cumulée de méthane produit en fonction du temps, ladite courbe est dite « courbe prédite » ;
d) on implémente au moins un modèle mathématique de digestion anaérobie sur la base d'au moins une des caractéristiques du substrat qui ont été prédites à l'étape b) à l'exception de celle du temps nécessaire pour la production de méthane exprimé en pourcentage de BMP du substrat de manière à déterminer une caractéristique de cinétique de dégradation dudit substrat pour laquelle on obtient à partir de ce modèle mathématique de digestion anaérobie une courbe de la quantité cumulée de méthane produit en fonction du temps présentant un écart minimal avec la courbe dite « courbe prédite » établie à l'étape c) ;
e) on implémente au moins un modèle mathématique de digestion anaérobie sur la base de la caractéristique de cinétique de dégradation dudit substrat déterminée à l'étape d) et d'au moins une des caractéristiques du substrat prédites à l'étape b) à l'exception de celle du temps nécessaire pour la production de méthane exprimé en pourcentage de BMP du substrat de façon à déterminer une estimation d'au moins une des caractéristiques de la biodégradation dudit substrat par digestion anaérobie dans un méthaniseur.

Les caractéristiques de la biodégradation d'un substrat par digestion anaérobie dans un méthaniseur peuvent être choisies parmi au moins l'une des caractéristiques suivantes :
- la production cumulée de méthane en fonction du temps par le méthaniseur;
- la composition du biogaz produit ;
- la concentration en produits intermédiaires et en inhibiteurs ;
- le pourcentage de DCO de substrat biodegradé.

Le substrat peut être choisi parmi ceux qui ont déjà été décrits ci-dessus, à savoir :
- les eaux usées industrielles, ainsi que les eaux usées urbaines qui, comme expliqué ci-dessus contiennent des particules solides ;
- les boues de stations d'épuration qui, comme expliqué ci-dessus contiennent des particules solides ;
- les micro-algues ;
- les déchets municipaux dont notamment les déchets alimentaires ;
- les déchets industriels ;
- les déchets de l'agriculture,
pris seuls ou en mélanges.

Dans un mode de réalisation de l'invention, le substrat contient des éléments sous forme solide.

Dans un mode de réalisation de l'invention, le substrat se présente sous forme particulaire, de préférence des particules de taille comprise entre 10 µm et 20 mm, encore plus préférentiellement entre 10 µm et 4 mm.

A l'étape a), les analyses de spectroscopie dans le NIR peuvent être réalisées sur l'échantillon de substrat qui a été au préalable broyé de manière à obtenir une poudre de cet échantillon de substrat. De manière encore plus avantageuse, l'échantillon de substrat peut avoir été lyophilisé, puis broyé. Préférentiellement, le diamètre de particule à l'issue du broyage est inférieur à 1 mm.

Bien entendu, l'homme du métier maîtrise les techniques de spectroscopie dans le NIR et saura comment mettre en forme de manière appropriée l'échantillon de substrat pour en acquérir ses données spectrales. Par exemple, il saura parfaitement mettre en oeuvre les étapes de lyophilisation et de broyage.

De manière avantageuse, chaque analyse de spectroscopie dans le NIR est répétée trois fois, chacune étant scannée indépendamment afin d'obtenir trois répétitions spectrales par échantillon de substrat pour pallier les potentiels problèmes d'homogénéité dudit échantillon. Dans un mode de réalisation de l'invention, chaque spectre NIR est lui-même la moyenne de 100 balayages afin de réduire le bruit. Le temps requis pour obtenir 100 balayages en d'environ 2 minutes. Les analyses par spectroscopie dans le NIR présentent l'avantage d'être très rapides.

Dans un mode de réalisation de l'invention, on traite avec au moins une méthode chimiométrique les données spectrales de l'étape a) de manière à obtenir des données spectrales traitées de l'échantillon de substrat.

Parmi les méthodes chimiométriques envisageables dans le cadre de la présente invention, on peut citer :
- le regroupement des trois répétitions pour chaque spectre en un spectre représentant la moyenne des trois spectres ;
- la transformation de la réflectance en absorbance: Absorbance=log(l/réflectance) ;
- la normalisation de variable aléatoire normale, ci-après abrégée normalisation « SNV » qui correspond à l'acronyme de sa dénomination anglophone « Standard Normal Variate » ;
- l'algorithme de Savitzky-Golay ;
- le detrending, à savoir la suppression de la ligne de base.

Le traitement des données spectrales par des méthodes chimiométriques est parfaitement à la portée de l'homme du métier. De plus, des outils informatiques pour traiter les spectres NIR sont à la disposition de l'homme du métier. On peut citer par exemple la boîte à outils FACT sous Scilab® 5.5.0

La caractéristique du substrat prédite à l'étape b) du temps nécessaire pour la production de méthane exprimé en pourcentage de BMP du substrat consiste en un ensemble de n valeurs des temps nécessaires pour la production de respectivement n pourcentages de BMP du substrat, n étant un nombre entier supérieur ou égal à 1, de préférence supérieur ou égal à 2. Ce nombre entier n peut être compris entre 1 et 100, plus préférentiellement entre 2 et 100. Par exemple, n peut être compris entre 5 et 19.

Par exemple, dans un mode de réalisation de l'invention, la caractéristique du temps nécessaire pour la production de méthane exprimé en pourcentage de BMP du substrat consiste en les 19 valeurs de temps nécessaires pour la production de respectivement 5% ; 10% ; 15% ; 20% ; 25% ; 30% ; 35% ; 40% ; 45%; 50%; 55%; 60%; 65%; 70%; 75%; 80%; 85%; 90% et 95% du BMP de l'échantillon de substrat. Le temps correspondant à la production de 100% du BMP n'est pas avantageux à prendre en compte, car il dépend essentiellement du temps d'expérimentation et n'est donc pas spécifique du substrat.

Le procédé d'estimation des caractéristiques de la biodégradation d'un substrat selon l'invention présente ainsi l'avantage qu'à partir d'une seule analyse de spectroscopie dans le NIR d'un échantillon de substrat et en ayant recours à un modèle de prédiction des caractéristiques d'un substrat, l'opérateur peut sur la base de ces caractéristiques du substrat implémenter un modèle de digestion anaérobie et ainsi déterminer une estimation des caractéristiques de la digestion anaérobie pour anticiper les performances de son méthaniseur.

Le procédé d'estimation des caractéristiques de la biodégradation d'un substrat selon l'invention est rapide et aisé à mettre en oeuvre, car il s'affranchit de toute analyse de laboratoire et les données spectrales par spectroscopie NIR sur un échantillon de substrat sec sont facilement et rapidement obtenables (en environ 2 minutes).

De plus, le procédé d'estimation des caractéristiques de la biodégradation d'un substrat selon l'invention présente une excellente justesse du fait de la très bonne fiabilité des prédictions des caractéristiques du substrat qui sont fournies par un modèle de prédiction des caractéristiques d'un substrat à partir des données spectrales dudit substrat.

En outre, le procédé d'estimation selon l'invention présente l'avantage que les caractéristiques de la biodégradation d'un substrat sont estimées à partir notamment de l'implémentation dans un modèle mathématique de digestion anaérobie de la caractéristique de cinétique de dégradation dudit substrat qui a elle-même été déterminée à l'étape d) grâce aux valeurs du BMP et à l'ensemble des n valeurs du temps nécessaire pour la production de méthane exprimé en pourcentage de BMP du substrat.

La détermination de la cinétique de dégradation du substrat présente une excellente fiabilité du fait qu'elle a été déterminée à partir d'une connaissance précise de la quantité cumulée de méthane produit en fonction du temps; cette connaissance résultant elle-même de la connaissance du BMP et d'un ensemble de n valeurs du temps nécessaire pour la production de méthane exprimé en pourcentage de BMP du substrat qui ont été prédites à l'étape b) à partir de données spectrales dans le NIR de l'échantillon de substrat. Plus le nombre n des valeurs prédites des temps nécessaires pour la production de méthane exprimé en pourcentage de BMP du substrat est élevé, plus l'estimation de la courbe de production du méthane en fonction du temps sera précise.

L'estimation précise de la cinétique de production de méthane permet en effet de calibrer avec finesse le modèle mathématique de digestion anaérobie qui permettra lui-même une juste estimation et anticipation des performances de production du méthaniseur dans le temps. Le fait de pouvoir anticiper à tout instant les performances permet à l'opérateur entre autre d'ajuster au mieux le temps de séjour ou autres paramètres opératoires de son unité. Cela lui permet aussi de travailler sur la recette d'alimentation qui maximisera ses performances dans les conditions de fonctionnement de l'unité.

Ainsi, grâce au procédé d'estimation selon l'invention, les performances d'un digesteur anaérobie en fonction des alimentations en substrats peuvent être estimées afin que l'opérateur ait la possibilité d'aisément optimiser les recettes d'alimentation de son méthaniseur, et ce dans un lapse de temps et à un coût très faibles qui correspondent aux exigences du domaine technique des unités de méthanisation. En effet, l'opérateur peut disposer de manière quasi instantanée d'une estimation des caractéristiques de la biodégradation par la digestion anaérobie du substrat qu'il compte incorporer dans le méthaniseur, et ce donc sans avoir à stocker ledit substrat le temps de l'obtention de cette estimation. On comprend ainsi tout l'intérêt du procédé d'estimation des caractéristiques de la biodégradation d'un substrat selon la présente invention.

De manière avantageuse, le procédé d'estimation selon l'invention est mis en oeuvre par une unité de traitement informatique.

Dans un mode de réalisation de l'invention, aux étapes d) et e), le modèle mathématique de digestion anaérobie est un modèle ADM1 ou l'une des variantes du modèle ADM1. Dans un autre mode de réalisation de l'invention, il peut s'agir du modèle mathématique de digestion anaérobie tel que décrit par exemple dans la publication Bernard, O. et al, Dynamical Model Development and Parameter Identification for an Anaerobic Wastewater Treatment Process, Biotechnology and Bioengineering, (2001), doi : 10.1002/bit.10036. Ces modèles mathématiques de digestion anaérobie sont parfaitement connus de l'homme du métier.

Dans un mode de réalisation de l'invention, à l'étape e), le modèle mathématique de digestion anaérobie est en outre implémenté sur la base d'au moins un paramètre de fonctionnement du méthaniseur. Les paramètres de fonctionnement du méthaniseur peuvent être choisis parmi :
- les microorganismes ;
- le pH ;
- la température ;
- le volume de liquide du méthaniseur ;
- le volume du ciel gazeux du méthaniseur ;
- le temps de séjour dans le méthaniseur ;
- les paramètres d'agitation du méthaniseur ;
- la teneur en matière sèche ;
- le débit de biogaz ;
- la composition du biogaz ;
- la concentration en acides gras volatils ;
- la concentration en inhibiteurs tels que l'ammoniaque.

Parmi ces paramètres de fonctionnement listés ci-dessus, certains peuvent être des paramètres de fonctionnement choisis par l'opérateur (autrement dit des paramètres de fonctionnement fixés par l'opérateur), d'autres peuvent être des paramètres qui découlent des choix de l'opérateur sur d'autres paramètres de fonctionnement et des caractéristiques du substrat biodégradé (autrement dit de la recette d'alimentation du méthaniseur).

De plus, certains de ces paramètres de fonctionnement peuvent évoluer au cours du temps. Ainsi, dans un mode de réalisation de l'invention, à l'étape e), le modèle mathématique de digestion anaérobie est implémenté en temps réel. Autrement dit, dans ce mode de réalisation de l'invention, le modèle mathématique de digestion anaérobie est en outre implémenté sur la base d'au moins un paramètre de fonctionnement du méthaniseur mesuré en temps réel au cours de la digestion anaérobie.

Ce mode de réalisation de l'invention est particulièrement avantageux car l'estimation des caractéristiques de la biodégradation du substrat par digestion anaérobie dans ledit méthaniseur est établie non seulement sur la base des caractéristiques dudit substrat, mais également sur la base de paramètres de fonctionnement du méthaniseur qui peuvent être notamment des paramètres de fonctionnement du méthaniseur mesurés en temps réel au cours de la digestion anaérobie.

A titre d'exemple, une telle implémentation d'un modèle mathématique de digestion anérobie est réalisée par le logiciel MeMo® commercialisé par la société BioEnTech.

Ainsi avec ces différents modes de réalisation du procédé d'estimation selon l'invention, et notamment lorque le procédé d'estimation est mis en oeuvre par une unité de traitement informatique, les différentes situations peuvent être envisagées :
1^{ière} situation : L'opérateur du méthaniseur ne dispose pas d'un dispositif d'acquisition de données spectrales dans le NIR de son substrat, ni de logiciel pour implémenter en temps réel un modèle mathématique de digestion anaérobie tel que le logiciel MeMo® :
   L'opérateur transmet un échantillon de son substrat à un laboratoire qui envoie les données spectrales de ce substrat qu'il aura acquises sur un serveur dédié. En retour, l'opérateur reçoit un accès lui permettant d'obtenir une estimation des caractéristiques de la biodégradation du substrat obtenue à partir d'un modèle mathématique de digestion anaérobie qui ne tient pas compte des paramètres de fonctionnement du méthaniseur en temps réel.
- 2^{ième} situation : L'opérateur du méthaniseur dispose d'un dispositif d'acquisition de données spectrales dans le NIR d'un échantillon de son substrat mais pas de logiciel pour implémenter en temps réel un modèle mathématique de digestion anaérobie tel que le logiciel MeMo®:
   L'opérateur acquiert des données spectrales d'un échantillon de son substrat, puis les envoie sur un serveur dédié. En retour, l'opérateur reçoit un accès lui permettant d'obtenir une estimation des caractéristiques de la biodégradation du substrat obtenue à partir d'un modèle mathématique de digestion anaérobie qui ne tient pas compte des paramètres de fonctionnement du méthaniseur en temps réel.
- 3^{ième} situation : L'opérateur du méthaniseur ne dispose pas d'un dispositif d'acquisition de données spectrales dans le NIR d'un échantillon de son substrat mais il dispose d'un logiciel pour implémenter en temps réel un modèle mathématique de digestion anaérobie tel que le logiciel MeMo® :
   L'opérateur transmet un échantillon de son substrat à un laboratoire qui envoie les données spectrales de ce substrat qu'il aura acquises sur un serveur dédié (par exemple l'interface MeMo®). Ces données spectrales sont intégrées au logiciel de manière à obtenir une estimation des caractéristiques de la biodégradation du substrat qui est établie en tenant également compte des paramètres de fonctionnement du méthaniseur en temps réel.
- 4^{ième} situation : L'opérateur du méthaniseur dispose d'un dispositif d'acquisition de données spectrales dans le NIR d'un échantillon de son substrat et d'un logiciel pour implémenter en temps réel un modèle mathématique de digestion anaérobie tel que le logiciel MeMo® :
   L'opérateur acquiert des données spectrales de son substrat qu'il envoie sur un serveur dédié (par exemple l'interface MeMo®). Ces données spectrales sont intégrées au logiciel et permettent d'obtenir une estimation des caractéristiques de la biodégradation du substrat qui est obtenue à partir d'un modèle mathématique de digestion anaérobie tenant compte des paramètres de fonctionnement du méthaniseur en temps réel.

L'étape souvent limitante est l'acheminement de l'échantillon à un laboratoire disposant d'un dispositif d'acquisition de données spectrales dans le NIR, ainsi que le temps de séchage de l'échantillon s'il n'est pas sec. Si l'opérateur souhaite analyser le plus rapidement possible ses substrats il est donc dans son intérêt de pouvoir disposer d'un dispositif d'acquisition de données spectrales dans le NIR à proximité.

Le procédé d'estimation selon l'invention peut comprendre une étape préalable au cours de laquelle on réalise un fractionnement chimique séquentiel du substrat. On obtient une pluralité de fractions de ce substrat. Le procédé d'estimation selon l'invention est alors mis en oeuvre à partir des données spectrales des échantillons de l'ensemble de ces fractions du substrat. En d'autres termes, dans ce mode de réalisation de l'invention, l'échantillon de substrat comprend les échantillons de substrats de chacune des fractions dudit substrat.

Par exemple, le substrat peut être fractionné en les 4 fractions suivantes :
- une fraction facilement extractible dite « fraction Xrc » ;
- une fraction modérément extractible «fraction Xmc » ;
- une fraction lentement extractible dite « fraction Xsc » ;
- une fraction non extractible dite « fraction Xne ».

Le fractionnement chimique séquentiel du substrat présente l'avantage d'améliorer la justesse de l'estimation des caractéristiques de la biodégradation dudit substrat par digestion anaérobie par comparaison à l'estimation qui aurait été obtenue en l'absence de ce fractionnement chimique séquentiel.

Si le substrat est fractionné chimiquement dans une étape préalable, cela peut allonger la durée du procédé d'estimation selon l'invention, mais en tous les cas dans une moindre mesure que si des analyses de laboratoire avaient été mises en oeuvre pour estimer les caractéristiques d'un substrat qui, rappelons-le, peuvent s'étaler sur plusieurs mois. En effet, la réalisation des fractionnements chimiques nécessite généralement quelques jours (environ au moins 4 jours).

Sans fractionnement chimique du substrat, l'obtention de l'estimation des caractéristiques de la biodégradation du substrat sec est quasi immédiate. Elle va dépendre essentiellement du temps d'acquisition des données spectrales du substrat. Ce temps d'acquisition est d'environ 2 minutes.

Ainsi, selon ses contraintes, dont notamment la durée de disponibilité du substrat (par exemple cette durée peut être liée aux impératifs de stockage du substrat) et selon le degré de justesse de l'estimation des caractéristiques de la biodégradation du substrat qu'il souhaite obtenir, l'opérateur pourra décider de réaliser ou non un fractionnement chimique séquentiel sur le substrat qu'il a l'intention d'incorporer dans un méthaniseur.

A l'étape b), les caractéristiques du substrat qui sont prédites peuvent être en outre au moins l'une des caractéristiques suivantes :
- la composition biochimique du substrat ;
- la DCO du substrat ;
- la biodégradabilité du substrat ;
- la fraction de matière organique dégradable ;
- l'accessibilité du substrat ;
- la biodégradabilité du substrat.

La composition biochimique du substrat peut comprendre au moins l'une des teneurs suivantes :
- la teneur en glucides du substrat ;
- la teneur en lipides du substrat ;
- la teneur en protéines du substrat.

Comme expliqué ci-dessus, un ratio de conversion permet d'obtenir la teneur en protéines à partir de la teneur en azote.

Dans un mode de réalisation de l'invention, au cours de l'étape d) du procédé d'estimation, on implémente le modèle de digestion anaérobie sur la base d'au moins le BMP et la composition biochimique du substrat qui ont été prédits à l'étape b).

Dans un mode de réalisation de l'invention, au cours de l'étape e) du procédé d'estimation, on implémente le modèle de digestion anaérobie sur la base de la caractéristique de la cinétique de dégradation du substrat qui a été déterminée à l'étape d), ainsi que d'au moins le BMP et la composition biochimique du substrat qui ont été prédits à l'étape b).

Un mode de réalisation de l'invention peut consister en la réalisation de l'étape d) et de l'étape e) de la manière telle que décrite juste ci-dessus.

Dans un mode de réalisation de l'invention, au cours de l'étape d) du procédé d'estimation, on implémente le modèle de digestion anaérobie sur la base d'au moins le BMP et d'au moins une des teneurs de la composition biochimique choisies parmi la teneur en glucides, lipides et protéines du substrat qui ont été prédits à l'étape b).

Dans un mode de réalisation de l'invention, au cours de l'étape e) du procédé d'estimation, on implémente le modèle de digestion anaérobie sur la base de la caractéristique de la cinétique dégradation du substrat qui a été déterminée à l'étape d), ainsi que d'au moins le BMP et d'au moins une des teneurs de la composition biochimique choisies parmi la teneur en glucides, lipides et protéines du substrat qui ont été prédits à l'étape b).

Un mode de réalisation de l'invention peut consister en la réalisation de l'étape d) et de l'étape e) de la manière telle que décrite juste ci-dessus.

Le procédé d'estimation selon l'invention peut comprendre une étape supplémentaire de conversion d'au moins une des caractéristiques du substrat qui ont été prédites à l'étape b). Le modèle mathématique de digestion anaérobie est alors implémenté sur la base des caractéristiques du substrat qui, le cas échéant, auront été converties.

Par exemple, dans un mode de réalisation de l'invention, l'étape de conversion peut être réalisée en multipliant la masse de lipides prédite du substrat par la DCO théorique d'un lipide prise à 2,87 gO₂/g de matière sèche, comme cela est expliqué dans la publication de Batstone et al. datant de 2002 qui a été mentionnée ci-dessus.

Par exemple, dans un mode de réalisation de l'invention, l'étape de conversion peut être réalisée en multipliant la teneur en azote prédite du substrat par 6,25. Ce ratio est communément utilisé en agro-alimentaire.

Dans un mode de réalisation de l'invention, à l'étape e) du procédé d'estimation selon l'invention, le modèle mathématique de digestion anaérobie peut être implémenté sur la base du BMP, de la biodégradabilité, de la teneur en glucides, ainsi que de la DCO qui auront été prédits à l'étape a), et ce sans étape de conversion de ces caractéristiques du substrat.

En ce qui concerne la DCO, si le substrat a été fractionné par fractionnement séquentiel chimique en une pluralité de frations, alors le modèle mathématique de digestion anaérobie peut être implémenté sur la base de la DCO prédite de chacune des fractions du substrat.

Dans un mode de réalisation de l'invention, si le substrat n'a pas été fractionné chimiquement, il se compose d'une seule fraction dite « fraction Xc », alors à l'étape e), le modèle mathématique de digestion anaérobie peut être implémenté sur la base des caractéristiques suivantes du substrat :
- la cinétique de dégradation ;
- la biodégradabilité ;
- la teneur en protéines ;
- la teneur en glucides ; et
- la teneur en lipides.

Dans un mode de réalisation de l'invention, si le substrat a été fractionné chimiquement en une pluralité de fractions, alors à l'étape e), le modèle mathématique de digestion anaérobie peut être implémenté sur la base des caractéristiques suivantes du substrat :
- le pourcentage de DCO correspondant à chaque fraction ;
- la cinétique de dégradation de chaque fraction ;
- la biodégradabilité de chaque fraction ;
- la teneur en protéines de chaque fraction ;
- la teneur en glucides de chaque fraction ; et
- la teneur en lipides de chaque fraction.

Dans un mode de réalisation de l'invention, le modèle de prédiction des caractéristiques d'un substrat destiné à être incorporé dans un méthaniseur pour être biodégradé par digestion anaérobie peut avoir été établi avec un procédé de configuration d'un modèle de prédiction tel que décrit ci-dessous.

Plus précisément, ce procédé de configuration d'un modèle de prédiction des caractéristiques d'un substrat repose sur la base de corrélations entre des données spectrales et différentes caractéristiques expérimentales d'échantillons de substrat déterminées par des analyses de laboratoire. Ce procédé de configuration d'un mode de prédiction présente une très bonne fiabilité et justesse qui confère ainsi au procédé d'estimation des caractéristiques de la biodégradation d'un substrat selon l'invention d'excellentes performances pour estimer ce qui va se produire au cours de la digestion anaérobie avec un substrat donné. Le procédé d'estimation selon l'invention fournit une estimation des caractéristiques de la biodégradation d'un substrat dans un méthaniseur qui tient dûment compte des caractéristiques de ce substrat.

Le procédé de configuration à partir duquel a été établi le modèle de prédiction des caractéristiques d'un substrat destiné à être incorporé dans un méthaniseur pour être biodégradé par digestion anaérobie qui est mis en oeuvre à l'étape b) du procédé d'estimation selon l'invention, peut comprendre au moins les étapes suivantes :
1) on dispose d'un ensemble d'échantillons de substrats d'un méthaniseur ;
2) on soumet ledit ensemble d'échantillons de substrats à une analyse de spectroscopie dans le NIR de manière à acquérir des données spectrales de chaque échantillon de substrat ;
3) on réalise des analyses de laboratoire déterminées sur chaque échantillon de substrat de manière à obtenir pour chaque échantillon de substrat au moins les caractéristiques expérimentales suivantes :
   - le BMP dudit échantillon de substrat,
   - le temps nécessaire pour la production de méthane exprimé en pourcentage de BMP de l'échantillon de substrat ;
4) on corrèle les données spectrales de l'étape 2) avec les caractéristiques expérimentales obtenues à l'étape 3) de manière à établir un modèle de prédiction qui est configuré pour prédire des caractéristiques d'un substrat destiné à être incorporé dans un méthaniseur à partir de données spectrales d'un échantillon de ce substrat.

Au cours de l'étape 4) du procédé de configuration, il est établi pour chaque caractéristique de substrat une corrélation entre les caractéristiques expérimentales de tous les échantillons de substrats qui sont associées à cette caractéristique de substrat et les données spectrales de tous les échantillons de substrat. En d'autres termes, le modèle de prédiction obtenu à l'issue de l'étape 4) a été établi sur la base de toutes les corrélations qui ont été déterminées entre les caractéristiques expérimentales de chaque caractéristique du substrat et les données spectrales. Cela confère une très bonne fiabilité et justesse au modèle de prédiction des caractéristiques d'un substrat.

A l'étape 1) du procédé, on peut augmenter le nombre d'échantillons de substrats dont on dispose en réalisant des mélanges d'échantillons de substrats entre eux de manière à obtenir des échantillons de substrats supplémentaires. En effet, plus le modèle de prédiction des caractéristiques d'un substrat aura été configuré avec un nombre élevé d'échantillons de substrats, meilleur sera sa justesse. En d'autres termes, il peut être avantageux si l'on dispose de peu d'échantillons de substrats d'en augmenter leur nombre par des mélanges entre eux de manière à ce que le modèle de prédiction soit établi à partir d'un nombre d'échantillons de substrats satisfaisant pour établir les corrélations les plus justes entre des données spectrales et des caractéristiques expérimentales desdits échantillons de substrats.

Si l'on crée des échantillons de substrats supplémentaires en effectuant des mélanges, il n'est pas nécessaire d'effectuer toutes les analyses de laboratoire de ces échantillons supplémentaires car certaines de leurs caractéristiques expérimentales peuvent être déduites des caractéristiques expérimentales des échantillons de substrats utilisés pour ces mélanges en tenant compte de leurs proportions. Par contre, il est nécessaire d'effectuer les analyses de spectroscopie de chacun de ces échantillons de substrats supplémentaires car leurs données spectrales ne peuvent pas être déduites de celles des échantillons de substrats dont ils sont issus.

A l'étape 2), les analyses de spectroscopie dans le NIR pour l'acquisition de données spectrales de chaque échantillon de substrat peuvent être réalisées sur chacun des échantillons de substrats de la même manière que cela a été décrit ci-dessus pour la description de l'étape a) du procédé d'estimation selon l'invention.

De manière avantageuse, chaque analyse de spectroscopie NIR est répétée trois fois, chacune étant scannée indépendamment afin d'obtenir trois répétitions spectrales par échantillon de substrat pour pallier les potentiels problèmes d'homogénéité dudit échantillon. Dans un mode de réalisation de l'invention, chaque spectre NIR est lui-même la moyenne de 100 balayages afin de réduire le bruit. Le temps requis pour obtenir 100 balayages en d'environ 2 minutes. Les analyses par spectroscopie NIR présentent l'avantage d'être très rapides.

Dans un mode de réalisation de l'invention, on traite avec au moins une méthode chimiométrique les données spectrales acquises à l'étape 2) de manière à obtenir des données spectrales traitées de chaque échantillon de substrat qu'on corrèle à l'étape 4) avec les caractéristiques expérimentales obtenues à l'étape 3) de chaque échantillon de substrat.

Les méthodes chimiométriques peuvent être choisies parmi celles qui ont été décrites ci-dessus pour la description de l'étape a) du procédé d'estimation selon l'invention.

Au cours de l'étape 3), la détermination expérimentale du BMP de chaque échantillon de substrat est parfaitement à la portée de l'homme du métier. Un exemple d'analyse de laboratoire pour déterminer le BMP d'un échantillon de substrat est décrit dans la publication de Garcia-Gen, S. et al, Kinetic modelling of anaerobic hydrolysis of solid wastes, including disintegradation processes. Waste Management. (2014) doi : 10.1016/j.wasman. 2014.10.012.

La caractéristique expérimentale du temps nécessaire pour la production de méthane exprimé en pourcentage de BMP de l'échantillon de substrat peut consister en un ensemble de n valeurs de temps nécessaires pour la production de respectivement n pourcentages du BMP de l'échantillon de substrat comme cela a été décrit ci-dessus pour la description de l'étape b) du procédé d'estimation selon l'invention.

Au cours de l'étape 3), on peut en outre obtenir pour chaque échantillon de substrat au moins l'une des caractéristiques expérimentales suivantes :
- la composition biochimique du substrat ;
- la DCO du substrat ;
- la biodégradabilité du substrat ;
- la fraction de matière organique dégradable ;
- l'accessibilité du substrat ;
- la biodégradabilité du substrat.

De manière avantageuse, la composition biochimique de l'échantillon de substrat comprend au moins l'une des teneurs suivantes :
- la teneur en glucides de l'échantillon de substrat ;
- la teneur en lipides de l'échantillon de substrat ;
- la teneur en protéines de l'échantillon de substrat.

Les exemples d'analyses de laboratoire qui ont été détaillés ci-dessus peuvent être mis en oeuvre au cours de l'étape 3) pour déterminer les caractéristiques expérimentales de chaque échantillon de substrat. Bien entendu, d'autres analyses de laboratoire qui sont à la portée de l'homme du métier peuvent également être réalisées pour déterminer les caractéristiques expérimentales des échantillons de substrats.

A l'étape 4) au cours de laquelle on établit les corrélations entre les données spectrales et les caractéristiques expérimentales pour chaque caractéristique du substrat, il peut être avantageux de séparer l'ensemble des échantillons de substrats en :
- un 1^{ier} lot d'échantillons de substrats qui est utilisé pour établir des corrélations entre les données spectrales et les caractéristiques expérimentales pour chaque caractéristique de substrat, le 1^{ier} lot est dit « lot d'apprentissage » ;
- un 2^{ième} lot d'échantillons de substrats qui est utilisé pour valider les corrélations établies avec le 1^{ier} lot pour toutes les caractéristiques de substrat, le 2^{ième} lot est dit « lot de validation ».

De cette manière, on réalise une validation des corrélations pour chaque caractéristique de substrat.

De manière préférée, à l'étape 4), on corrèle les données spectrales de l'étape 2) avec les caractéristiques expérimentales obtenues à l'étape 3) avec au moins un traitement mathématique.

De manière tout à fait préférée, ledit traitement mathématique consiste en une régression des moindres carrés partiels (ci-après abrégé « PLS »).

La régression PLS est un traitement mathématique particulièrement approprié car elle maximise la covariance entre deux ensembles de données en recherchant les combinaisons linéaires des variables à partir de ses deux ensembles. Ces combinaisons linéaires sont appelées variables latentes. Les vecteurs de pondération qui sont utilisés pour calculer les combinaisons linéaires sont appelés vecteurs propres. Les variables latentes et les vecteurs propres viennent par paires. Les composantes de la régression PLS (à savoir des variables latentes) sont des combinaisons linéaires des variables initiales. Toutefois, les coefficients qui définissent ces composantes ne sont pas linéaires comme ils sont résolus par des régressions locales successives sur les variables latentes.

Par exemple, le traitement de régression PLS est réalisé avec la boîte à outils FACT sous Scilab® 5.5.0.

Le traitement de régression PLS est réalisé de manière indépendante pour chaque caractéristique du substrat. Cela signifie que le traitement de régression PLS utilisé pour une 1^{ière} caractéristique du substrat est indépendant, et donc susceptible d'être différent, de celui utilisé pour une 2^{ième} caractéristique du substrat.

Avantageusement, lors du traitement mathématique avec une régression PLS, pour chaque caractéristique du substrat, on réalise une validation croisée dont le résultat fournit des indicateurs sur la précision de la corrélation entre les données spectrales et les caractéristiques expérimentales associées à ladite caractéristique de substrat.

Dans le mode de réalisation de l'invention dans lequel on a séparé les échantillons de substrats en un 1^{ier} lot d'apprentissage et un 2^{ième} lot de validation et l'on effectue les corrélations avec une régression PLS, les corrélations établies avec le 1^{ier} lot d'apprentissage sont validées si les corrélations établies sur le 1^{ier} lot en conservant le même nombre de variables latentes et les mêmes paramètres de régression PLS fournissent sur le 2^{ième} lot de validation des résultats satisfaisants. Par résultats satisfaisants, on entend une faible différence entre, d'une part, les valeurs prédites en utilisant le modèle PLS calibré sur le 1^{ier} lot d'apprentissage et les spectres du 2^{ième} lot de validation et d'autre part, les valeurs expérimentales de l'étape b) du 2^{ième} lot de validation.

L'invention sera bien comprise à l'aide de la description détaillée qui suit, en référence au dessin schématique annexé représentant des résultats expérimentaux liés à l'invention.

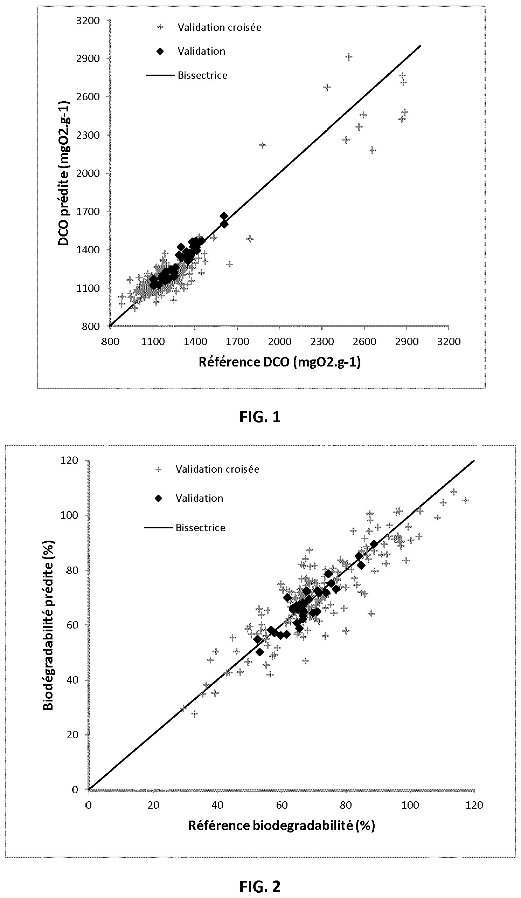

La figure 1 est un graphe représentant :
   - les résultats de la validation croisée pour la caractéristique de la DCO qui a été réalisée sur la base d'un 1^{ier} lot d'échantillons de substrats ;
   - les résultats de la validation des corrélations établies pour la caractéristique de la DCO avec le 1^{ier} lot d'échantillons de substrats à partir d'un 2^{ième} lot d'échantillons de substrats.
La figure 2 est un graphe représentant :
   - les résultats de la validation croisée pour la caractéristique de la biodégradabilité qui a été réalisée sur la base d'un 1^{ier} lot d'échantillons de substrats ;
   - les résultats de la validation des corrélations établies pour la caractéristique de la biodégradabilité avec le 1^{ier} lot d'échantillons de substrats à partir d'un 2^{ième} lot d'échantillons de substrats.

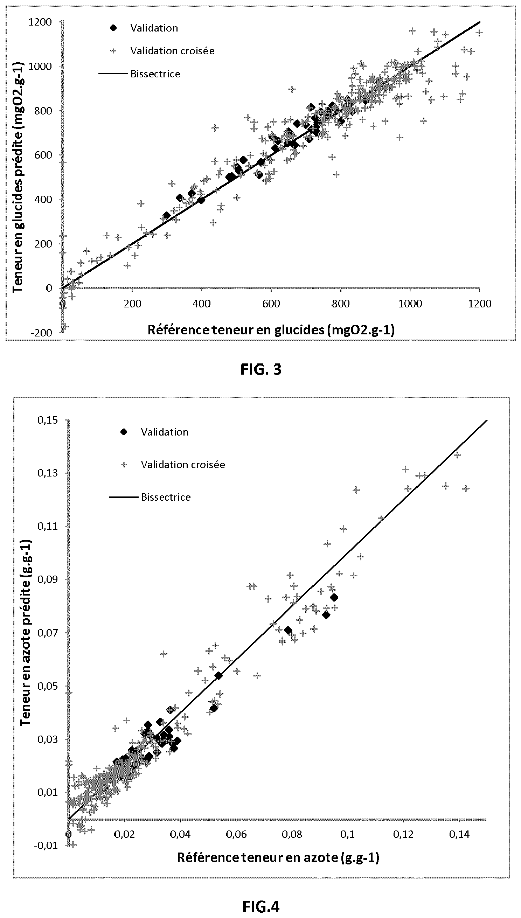

La figure 3 est un graphe représentant :
   - les résultats de la validation croisée pour la caractéristique de la teneur en glucides qui a été réalisée sur la base d'un 1^{ier} lot d'échantillons de substrats ;
   - les résultats de la validation des corrélations établies pour la caractéristique de la teneur en glucides avec le 1^{ier} lot d'échantillons de substrats à partir d'un 2^{ième} lot d'échantillons de substrats.
La figure 4 est un graphe représentant :
   - les résultats de la validation croisée pour la caractéristique de la teneur en azote qui a été réalisée sur la base d'un 1^{ier} lot d'échantillons de substrats ;
   - les résultats de la validation des corrélations établies pour la caractéristique de la teneur en azote avec le 1^{ier} lot d'échantillons de substrats à partir d'un 2^{ième} lot d'échantillons de substrats.

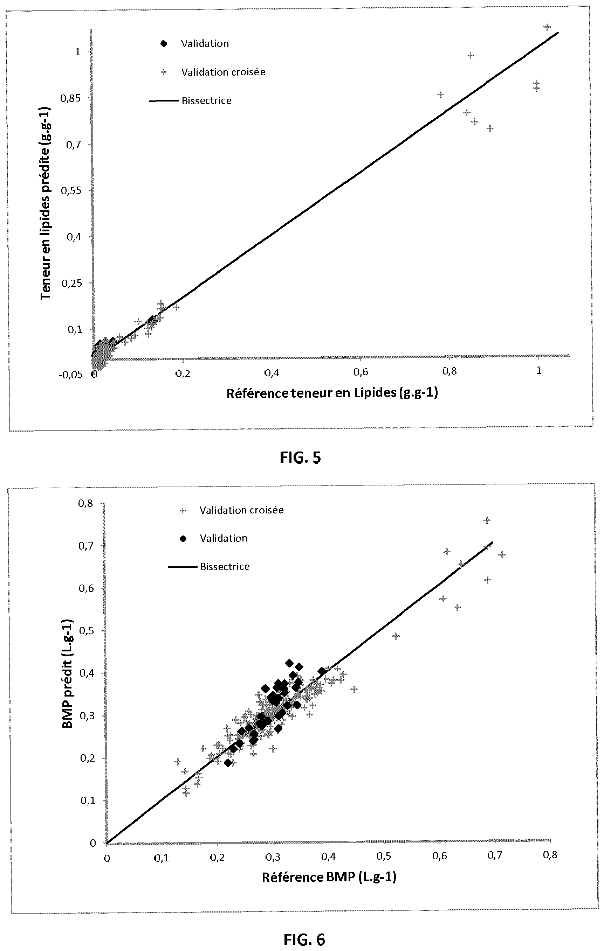

La figure 5 est un graphe représentant :
   - les résultats de la validation croisée pour la caractéristique de la teneur en lipides qui a été réalisée sur la base d'un 1^{ier} lot d'échantillons de substrats ;
   - les résultats de la validation des corrélations établies pour la caractéristique de la teneur en lipides avec le 1^{ier} lot d'échantillons de substrats à partir d'un 2^{ième} lot d'échantillons de substrats.
La figure 6 est un graphe représentant :
   - les résultats de la validation croisée pour la caractéristique du BMP qui a été réalisée sur la base d'un 1^{ier} lot d'échantillons de substrats ;
   - les résultats de la validation des corrélations établies pour la caractéristique du BMP avec le 1^{ier} lot d'échantillons de substrats à partir d'un 2^{ième} lot d'échantillons de substrats.

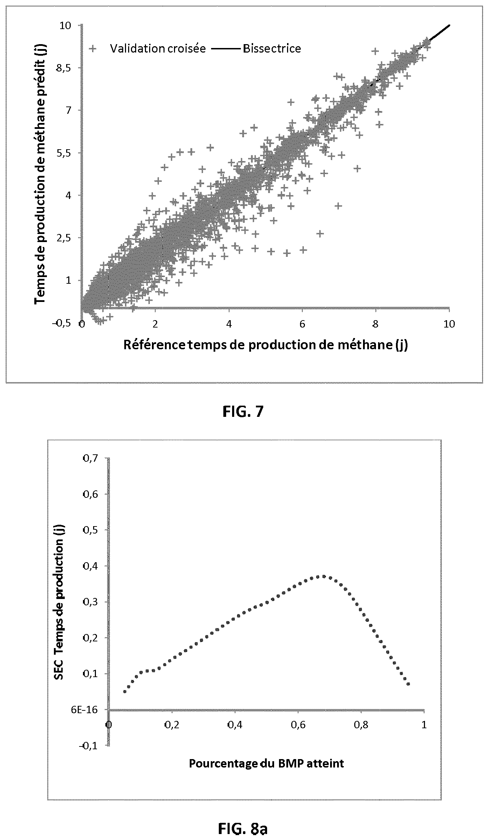

La figure 7 est un graphe représentant les résultats de la validation croisée pour la caractéristique du temps nécessaire pour la production de méthane exprimé en pourcentage de BMP.
La figure 8a est un graphe représentant l'écart type de calibration (ci-après abrégé « SEC » car provenant de l'acronyme anglophone pour « Standard of Error Calibration ») des résultats de la validation croisée pour la caractéristique du temps nécessaire pour la production de méthane exprimé en pourcentage de BMP.

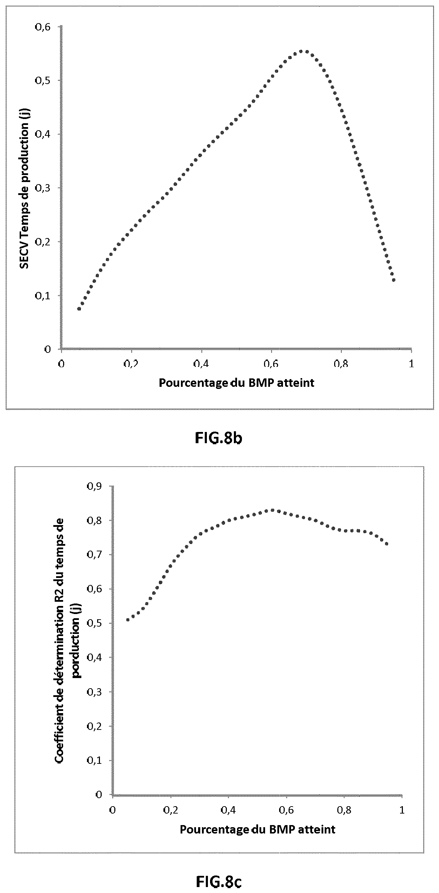

La figure 8b est un graphe représentant l'écart type de validation croisée (ci-après abrégé « SECV » car provenant de l'acronyme anglophone pour « Standard Error of Cross Validation »)des résultats de la validation croisée pour la caractéristique du temps nécessaire pour la production de méthane exprimé en pourcentage de BMP.
La figure 8c est un graphe représentant le coefficient R² de détermination entre la variable dépendante et la prédiction de cette variable des résultats de la validation croisée pour la caractéristique du temps nécessaire pour la production de méthane exprimé en pourcentage de BMP.

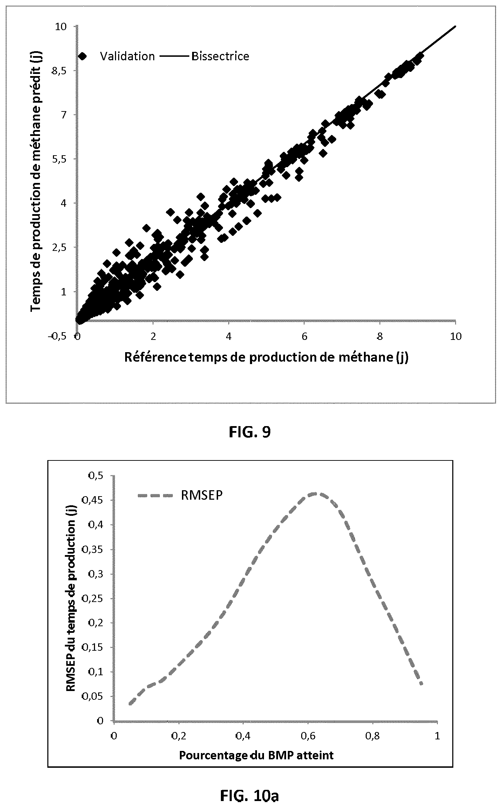

La figure 9 est un graphe représentant les résultats de la validation des corrélations établies pour la caractéristique du temps nécessaire pour la production de méthane exprimé en pourcentage de BMP avec le 1^{ier} lot d'échantillons de substrats à partir d'un 2^{ième} lot d'échantillons de substrats.
La figure 10a est un graphe représentant la racine carrée de l'erreur quadratique de prédiction (ci-après abrégée « RMSEP » car provenant de l'acronyme anglophone pour « Root Mean Standard Error of Prédiction ») des résultats de la validation des corrélations établies pour la caractéristique du temps nécessaire pour la production de méthane exprimé en pourcentage de BMP avec le 1^{ier} lot d'échantillons de substrats à partir d'un 2^{ième} lot d'échantillons de substrats.

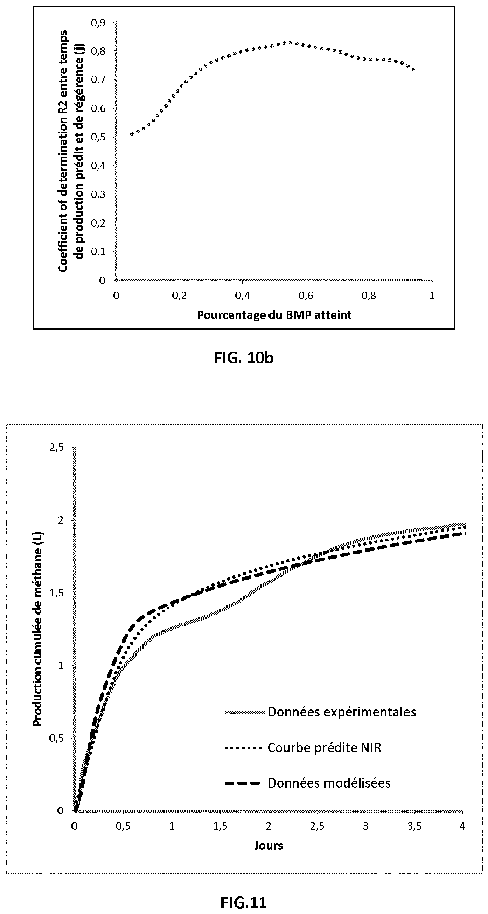

La figure 10b est un graphe représentant le coefficient R² de détermination entre la variable dépendante et la prédiction de cette variable des résultats de la validation des corrélations établies pour la caractéristique du temps nécessaire pour la production de méthane exprimé en pourcentage de BMP avec le 1^{ier} lot d'échantillons de substrats à partir d'un 2^{ième} lot d'échantillons de substrats.
La figure 11 est un graphe représentant trois courbes de production cumulée de méthane en fonction du temps.

### PARTIE EXPERIMENTALE :

### Description des substrats étudiés :

Les 31 substrats suivants ont été choisis pour les expérimentations qui sont détaillées ci-dessous : ananas, banane, carotte, chou vert, chou-fleur, courgette, fumier bovin, fumier ovin, fumier caprin, fumier équin, gras de porc, herbe, huile de table, lieu noir, mangue, micro-algues, nourriture pour cochon d'Inde, orange, paille, pain, pâte cuite, pêche, pomme, pomme de terre, pulpe de betterave, raisin, riz cuit, salade, viande, tomate et tourteaux de biodiesel à partir de tournesol.

### Analyses de laboratoire des échantillons de substrats :

Sur des échantillons de ces 31 substrats, on a réalisé des analyses de laboratoire afin de déterminer pour chacun de ces substrats :
- la teneur en glucides ;
- la teneur en azote ;
- la teneur en lipides ;
- la DCO ;
- la teneur de matières sèches (abrégé ci-après « MS ») qui correspond au résidu d'un substrat qui est obtenu après un séchage pendant 24 heures à 105°C.

De plus, ces 31 substrats ont été fractionnés chimiquement afin d'obtenir pour chacun d'eux :
- une fraction contenant les matières extraites lors de l'extraction avec un solvant contenant un mélange de soude à une concentration de 0,01 mol/L et de chlorure de sodium de 0,4 g/L : matières « REOM » ;
- une fraction contenant les matières extraites lors de l'extraction avec un solvant contenant un mélange de soude à une concentration de 0,1 mol/L : matières « MEOM » ;
- les matières extraites lors de l'extraction avec un solvant contenant de l'acide sulfurique concentré à 72% en volume : matières « SEOM » ;
- les matières restantes après ces extractions : matières « NEOM ».

Les analyses de laboratoire détaillées ci-dessus ont été réalisées sur chacun des 31 culots de :
- MEOM, SEOM et NEOM
- SEOM+NEOM.

Le culot NEOM n'a pas été analysé, car il était altéré par l'extraction à l'acide sulfurique et était trop opaque pour des analyses de spectroscopie dans le NIR.

On a obtenu un total de 93 analyses de laboratoire de teneur en glucides, azote, lipides, matières sèches et DCO. Les analyses de lipides n'ont pas été réalisées sur les culots, car cette analyse nécessite une quantité de matière trop importante qui n'était pas disponible dans les culots. De plus, les lipides ont été extraits dans les premières fractions.

### Détermination expérimentale du BMP des échantillons de substrats :

La détermination expérimentale du BMP des échantillons de substrats a été réalisée selon la méthode décrite dans la publication de Garcia-Gen, S. et al, Kinetic modelling of anaerobic hydrolysis of solid wastes, including disintegradation processes. Waste Management. (2014) Doi : 10.1016/j.wasman. 2014.10.012.

### Caractéristique expérimentale du temps nécessaire pour la production de méthane exprimé en pourcentage de BMP de l'échantillon de substrat :

La caractéristique expérimentale du temps nécessaire pour la production de méthane exprimé en pourcentage de BMP de l'échantillon de substrat consiste en les 19 valeurs de temps nécessaires pour la production de respectivement 5% ; 10% ; 15% ; 20% ; 25% ; 30% ; 35% ; 40% ; 45% ; 50% ; 55% ; 60% ; 65% ; 70% ; 75% ; 80% ; 85% ; 90% et 95% du BMP expérimental de chaque échantillon de substrat.

### Analyses de spectroscopie dans le NIR des échantillons de substrats :

Les 31 substrats et chacun des culots MEOM+SEOM+NEOM et SEOM+NEOM ont été lyophilisés, puis broyés à un diamètre de particule inférieur à 1 mm. Les poudres résultant du broyage ont ensuite été analysées par spectroscopie NIR en réflectance dans une gamme de longueur d'onde de 1000-2500 nm, avec une résolution de 2,5 x 106 nm, en utilisant un spectrophotomètre commercialisé par la société BUCHI sous la dénomination commerciale « BUCHI NIR Flex N-500 » et qui était équipé de l'accessoire solide 6 flacons.

Chaque analyse a été répétée trois fois, chacune étant scannée indépendamment afin d'obtenir trois répétitions spectrales par échantillon pour pallier les problèmes d'homogénéité de l'échantillon de substrat. Chaque spectre NIR était lui-même la moyenne de 100 balayages afin de réduire le bruit. Le temps requis pour obtenir 100 balayages était d'environ 2 minutes.

### Traitement des spectres NIR :

Des méthodes chimiométriques ont été utilisées pour traiter les données spectrales des échantillons de substrats.

Les traitements mathématiques suivants ont été effectués sur les spectres NIR des 279 spectres NIR des 31 échantillons de substrat et de leurs culots, et ce dans l'ordre ci-dessous:

### Traitement 1 :

- le regroupement des trois répétitions pour chaque spectre en un spectre représentant la moyenne des trois spectres ;
- la transformation de la réflectance en absorbance: Absorbance=log(l/réflectance) ;
- la normalisation SNV.

### Traitement 2 :

- le regroupement des trois répétitions pour chaque spectre en un spectre représentant la moyenne des trois spectres ;
- la transformation de la réflectance en l'absorbance: Absorbance=log(réflectance) ;
- normalization SNV ;
- l'algorithme de Savitzky-Golay.

### Traitement 3

- le regroupement des trois répétitions pour chaque spectre en un spectre représentant la moyenne des trois ;
- la transformation de la réflectance en l'absorbance: Absorbance=log(réflectance) ;
- la normalization SNV ;
- le detrending.

Pour réaliser le traitement des spectres NIR, la boîte à outils FACT sous Scilab® 5.5.0 a été utilisée.

De plus, afin d'augmenter le nombre de données spectrales, des substrats non extraits ont été mélangés entre eux.

On a ainsi obtenu pour :
- l'analyse de la teneur en azote : 334 caractéristiques expérimentales et 1002 spectres NIR associés qui ont été traités avec le traitement 2 ;
- l'analyse de la teneur en sucres : 334 caractéristiques expérimentales et 1002 spectres NIR associés qui ont été traités avec le traitement 2 ;
- l'analyse de la teneur en lipides : 209 caractéristiques expérimentales et 627 spectres NIR associés qui ont été traités avec le traitement 1 ;
- l'analyse de la DCO: 334 caractéristiques expérimentales et 1002 spectres NIR associés qui ont été traités avec le traitement 3 ;
- l'analyse de la biodégradabilité: 206 caractéristiques expérimentales et 618 spectres associés NIR qui ont été traités avec le traitement 2 ;
- l'analyse du BMP: 206 caractéristiques expérimentales et 618 spectres associés qui ont été traités avec le traitement 2 ;
- l'analyse du temps nécessaire pour la production de méthane exprimé en pourcentage de BMP : 242 x 19 caractéristiques expérimentales et 726 spectres associés qui ont été traités avec le traitement 2.

Sur l'ensemble des 31 échantillons de substrats, deux substrats, ainsi que leurs culots et les échantillons mélangés contenant une partie de ces deux substrats ont été isolés pour constituer un 2^{ième} lot dit « lot de validation » utilisé pour valider les corrélations établies avec le 1^{ier} lot dit « lot d'apprentissage » qui est donc constitué du reste des échantillons de substrats.

Le 1^{ier} lot d'échantillons de substrats a donc été utilisé pour établir des corrélations entre les données spectrales et les caractéristiques expérimentales pour chaque caractéristique de substrat de la manière suivante :
- analyse de la teneur en azote : 291 caractéristiques expérimentales et 873 spectres associés qui ont été traités avec le traitement 2 ;
- analyse de la teneur en sucre : 291 caractéristiques expérimentales et 873 spectres associés qui ont été traités avec le traitement 2 ;
- analyse de la teneur en lipides : 173 caractéristiques expérimentales et 519 spectres NIR associés qui ont été traités avec le traitement 1,
- analyse de la DCO: 291 caractéristiques expérimentales et 873 spectres associés qui ont été traités avec le traitement 3,
- analyse du BMP: 170 caractéristiques expérimentales et 510 spectres associés qui ont été traités avec le traitement 2,
- analyse de la biodégradabilité: 170 caractéristiques expérimentales et 510 spectres associés qui ont été traités avec le traitement 2,
- analyse du temps nécessaire pour la production de méthane exprimé en pourcentage de BMP: 207 x 19 caractéristiques expérimentales et 621 spectres associés qui ont été traités avec le traitement 2.

A partir des données spectrales et des caractéristiques expérimentales du lot d'apprentissage, un traitement informatique consistant en une régression PLS a été appliqué afin d'obtenir une corrélation entre les spectres NIR et les caractéristiques expérimentales pour chacune des caractéristiques du substrat.

Pour réaliser ce traitement mathématique de régression PLS, la boîte à outils FACT sous Scilab® 5.5.0 a été utilisée.

Les valeurs prédites en validation croisée étaient proches des valeurs de référence, la validité des corrélations entre les données spectrales et les caractéristiques expérimentales pour chaque caractéristique de substrat a ensuite été vérifiée avec le lot de validation.

Le lot de validation d'échantillons de substrats a donc été utilisé pour valider les corrélations établies entre les données spectrales et les caractéristiques expérimentales pour chaque caractéristique de substrat de la manière suivante :
- analyse de la teneur en azote : 43 données de référence et 129 spectres associés (Traitement 2) ;
- analyse de la teneur en sucre : 43 données de référence et 129 spectres associés (Traitement 2) ;
- analyse de la teneur en lipides : 36 données de référence et 108 spectres associés (Traitement 1) ;
- analyse de la DCO: 43 données de référence et 129 spectres associés (Traitement 3) ;
- analyse du BMP: 36 données de référence et 108 spectres associés (Traitement 2) ;
- analyse de la biodégradabilité: 36 données de référence et 108 spectres associés (Traitement 2) ;
- analyse du temps nécessaire pour la production de méthane exprimé en pourcentage de BMP: 35 données de référence et 105 spectres associés (Traitement 2).

Avec le lot de validation d'échantillons de substrats, les corrélations établies avec le lot d'apprentissage pour toutes les caractéristiques de substrat ont bien été validées.

Le graphe de la figure 1 représente les résultats de la validation croisée pour la caractéristique de DCO qui a été réalisée sur la base du 1^{ier} lot d'échantillons de substrats (lot d'apprentissage). Il s'agit des points notés « validation croisée ». En abscisse, il s'agit des caractéristiques expérimentales de la DCO (à savoir « Référence DCO ») du lot d'apprentissage et en ordonnée, il s'agit des valeurs de la DCO prédites grâce aux corrélations établies entres les caractéristiques expérimentales de la DCO et les données spectrales du lot d'apprentissage.

Pour cette validation croisée :
- le SEC est de 82 mgO₂/g de MS ;
- le SECV est de 99 mgO₂/g de MS ;
- le R² est 0,89.

Le graphe de la figure 1 représente en outre les résultats de la validation des corrélations établies pour la caractéristique de la DCO avec le 1^{ier} lot d'échantillons de substrats à partir du 2^{ième} lot d'échantillons de substrats (lot de validation). Il s'agit des points notés « validation ». En abscisse, il s'agit des caractéristiques expérimentales de la DCO (à savoir « Référence DCO ») du lot de validation et en ordonnée, il s'agit des valeurs de la DCO prédites grâce aux corrélations établies entres les caractéristiques expérimentales de la DCO et les données spectrales du lot de validation.

Pour cette validation :
- le R² est de 0,91 ;
- le RMSEP (à savoir la racine carrée de l'erreur quadratique de prédiction) est de 41 mgO₂/g de MS.

Au vu du graphe 1, on relève que pour la DCO, la prédiction des valeurs est correcte.

Le graphe de la figure 2 représente les résultats de la validation croisée pour la caractéristique de biodégradabilité qui a été réalisée sur la base du 1^{ier} ot d'échantillons de substrats (lot d'apprentissage). Il s'agit des points notés « validation croisée ». En abscisse, il s'agit des caractéristiques expérimentales de la biodéradabilité (à savoir « Référence biodégradabilité ») du lot d'apprentissage et en ordonnée, il s'agit des valeurs de la biodégradabilité prédites grâce aux corrélations établies entres les caractéristiques expérimentales de la biodégradabilité et les données spectrales du lot d'apprentissage.

Pour cette validation croisée :
- le SEC est de 6,2% ;
- le SECV est de 7,5% ;
- le R² est 0,78.

Le graphe de la figure 2 représente en outre les résultats de la validation des corrélations établies pour la caractéristique de la biodégradabilité avec le 1^{ier} lot d'échantillons de substrats à partir du 2^{ième} lot d'échantillons de substrats (lot de validation). Il s'agit des points notés « validation ». En abscisse, il s'agit des caractéristiques expérimentales de la biodégradabilité (à savoir « Référence biodégradabilité ») du lot de validation et en ordonnée, il s'agit des valeurs de la biodégradabilité prédites grâce aux corrélations établies entres les caractéristiques expérimentales de la biodégradabilité et les données spectrales du lot de validation.

Pour cette validation :
- le R² est de 0,83 ;
- le RMSEP est de 3,3 %.

Le graphe de la figure 3 représente les résultats de la validation croisée pour la caractéristique de la teneur en glucides qui a été réalisée sur la base du 1^{ier} lot d'échantillons de substrats (lot d'apprentissage). Il s'agit des points notés « validation croisée ». En abscisse, il s'agit des caractéristiques expérimentales de la teneur en glucides (à savoir « Référence teneur en glucides ») du lot d'apprentissage et en ordonnée, il s'agit des valeurs de la teneur en glucides prédites grâce aux corrélations établies entres les caractéristiques expérimentales de la teneur en glucides et les données spectrales du lot d'apprentissage.

Pour cette validation croisée :
- le SEC est de 77 mgO₂/g de MS ;
- le SECV est de 90 mgO₂/g de MS ;
- le R² est 0,90.

Le graphe de la figure 3 représente en outre les résultats de la validation des corrélations établies pour la caractéristique de la teneur en glicides avec le 1^{ier} lot d'échantillons de substrats à partir du 2^{ième} lot d'échantillons de substrats (lot de validation). Il s'agit des points notés « validation ». En abscisse, il s'agit des caractéristiques expérimentales de la teneur en glucides (à savoir « Référence teneur en glucides ») du lot de validation et en ordonnée, il s'agit des valeurs de la teneur en glucides prédites grâce aux corrélations établies entres les caractéristiques expérimentales de la teneur en glucides et les données spectrales du lot de validation.

Pour cette validation :
- le R² est de 0,95 ;
- le RMSEP est de 39,9 mgO₂/g de MS.

Le graphe de la figure 4 représente les résultats de la validation croisée pour la caractéristique de la teneur en azote qui a été réalisée sur la base du 1^{ier} lot d'échantillons de substrats (lot d'apprentissage). Il s'agit des points notés « validation croisée ». En abscisse, il s'agit des caractéristiques expérimentales de la teneur en azote (à savoir « Référence teneur en azote ») du lot d'apprentissage et en ordonnée, il s'agit des valeurs de la teneur en azote prédites grâce aux corrélations établies entres les caractéristiques expérimentales de la teneur en azote et les données spectrales du lot d'apprentissage.

Pour cette validation croisée :
- le SEC est de 5,5 10⁻³ g/g ;
- le SECV est 7,2 10⁻³ g/g ;
- le R² est 0,94.

Le graphe de la figure 4 représente en outre les résultats de la validation des corrélations établies pour la caractéristique de la teneur en azote avec le 1^{ier} lot d'échantillons de substrats à partir du 2^{ième} lot d'échantillons de substrats (lot de validation). Il s'agit des points notés « validation ». En abscisse, il s'agit des caractéristiques expérimentales de la teneur en azote (à savoir « Référence teneur en azote ») du lot de validation et en ordonnée, il s'agit des valeurs de la teneur en azote prédites grâce aux corrélations établies entres les caractéristiques expérimentales de la teneur en azote et les données spectrales du lot de validation.

Pour cette validation :
- le R² est de 0,92 ;
- le RMSEP est de 5,6 10⁻³ g/g.

Le graphe de la figure 5 représente les résultats de la validation croisée pour la caractéristique de la teneur en lipides qui a été réalisée sur la base du 1^{ier} lot d'échantillons de substrats (lot d'apprentissage). Il s'agit des points notés « validation croisée ». En abscisse, il s'agit des caractéristiques expérimentales de la teneur en lipides (à savoir « Référence teneur en lipides ») du lot d'apprentissage et en ordonnée, il s'agit des valeurs de la teneur en lipides prédites grâce aux corrélations établies entres les caractéristiques expérimentales de la teneur en lipides et les données spectrales du lot d'apprentissage.

Pour cette validation croisée :
- le SEC est de 1,6 10⁻² g/g ;
- le SECV est 2,5 10⁻² g/g ;
- le R² est 0,98.

Le graphe de la figure 5 représente en outre les résultats de la validation des corrélations établies pour la caractéristique de la teneur en lipides avec le 1^{ier} lot d'échantillons de substrats à partir du 2^{ième} lot d'échantillons de substrats (lot de validation). Il s'agit des points notés « validation ». En abscisse, il s'agit des caractéristiques expérimentales de la teneur en lipides (à savoir « Référence teneur en lipides ») du lot de validation et en ordonnée, il s'agit des valeurs de la teneur en lipides prédites grâce aux corrélations établies entres les caractéristiques expérimentales de la teneur en lipides et les données spectrales du lot de validation.

Pour cette validation :
- le R² est de 0,79 ;
- le RMSEP est de 1,46 10⁻² g/g.

Le graphe de la figure 6 représente les résultats de la validation croisée pour la caractéristique de la teneur en lipides qui a été réalisée sur la base du 1^{ier} lot d'échantillons de substrats (lot d'apprentissage). Il s'agit des points notés « validation croisée ». En abscisse, il s'agit des caractéristiques expérimentales de la teneur en lipides (à savoir « Référence BMP ») du lot d'apprentissage et en ordonnée, il s'agit des valeurs du BMP prédites grâce aux corrélations établies entres les caractéristiques expérimentales du BMP et les données spectrales du lot d'apprentissage.

Pour cette validation croisée :
- le SEC est de 0,0201 L/g ;
- le SECV est 0,029 L/g ;
- le R² est 0,91.

Le graphe de la figure 6 représente en outre les résultats de la validation des corrélations établies pour la caractéristique du BMP avec le 1^{ier} lot d'échantillons de substrats à partir du 2^{ième} lot d'échantillons de substrats (lot de validation). Il s'agit des points notés « validation ». En abscisse, il s'agit des caractéristiques expérimentales du BMP (à savoir « Référence BMP ») du lot de validation et en ordonnée, il s'agit des valeurs du BMP prédites grâce aux corrélations établies entres les caractéristiques expérimentales du BMP et les données spectrales du lot de validation.

Pour cette validation :
- le R² est de 0,71 ;
- le RMSEP est de 0,038 L CH₄/g MS.

Les résultats détaillés ci-dessus témoignent que la prédiction de la teneur en azote, la teneur en glucides et de la teneur en lipide sont extrêmement précises aussi bien pendant les étapes de validations croisées que de validations. Cela valide les modèles de régression PLS établis.

Il en est de même pour la caractéristique de la DCO qui présente toutefois une imprécision un peu plus élevée que celle des autres caractéristiques. Cette imprécision légèrement plus importante peut s'expliquer par la certaine imprécision des caractéristiques expérimentales de la DCO qui ont été obtenues à partir du kit commercial utilisé pour la détermination expérimentale de la DCO.

La prédiction du BMP présente une RMSEP de 0,038 L CH₄/g MS. Cela peut s'expliquer par le fait que les caractéristiques expérimentales du BMP ont été obtenues à partir d'expériences biologiques dont l'erreur de mesure expérimentale peut être plus élevée que celle d'analyses chimiques (telles que celles utilisées pour la détermination de la composition biochimique du substrat). Cette erreur de mesure expérimentale pour le BMP peut être d'environ 20%. Par conséquent, cette imprécision expérimentale quant à la caractéristique du BMP a des répercussions sur les prédictions obtenues avec la spectroscopie dans le NIR. Malgré cette imprécision quant à la caractéristique expérimentale du BMP dont on ne peut s'affranchir du fait que sa détermination repose sur des expériences biologiques, il n'en demeure pas moins que les présents résultats expérimentaux témoignent d'une prédiction du BMP qui est fiable et parfaitement exploitable à des fins industrielles.

La biodégradabilité étant calculée par le rapport : BMP/(350 x DCO), les erreurs expérimentales de la DCO et du BMP détaillées ci-dessus ont inévitablement des répercussions sur la prédiction de la biodégradabilité. On relève cependant de très bonnes performances du modèle de régression PLS établi.

La figure 7 est un graphe représentant les résultats de la validation croisée pour la caractéristique du temps nécessaire pour la production de méthane exprimé en pourcentage de BMP. La figure 8a est un graphe représentant le SEC des résultats de la validation croisée pour cette caractéristique du substrat. La figure 8b est un graphe représentant le SECV des résultats de la validation croisée pour cette caractéristique du substrat. La figure 8c est un graphe représentant le coefficient R² des résultats de la validation croisée pour cette caractéristique du substrat.

La figure 9 est un graphe représentant les résultats de la validation des corrélations établies pour la caractéristique du temps nécessaire pour la production de méthane exprimé en pourcentage de BMP avec le 1^{ier} lot d'échantillons de substrats à partir du 2^{ième} lot d'échantillons de substrats. La figure 10a est un graphe représentant la RMSEP des résultats de la validation des corrélations établies pour cette caractéristique avec le 1^{ier} lot d'échantillons de substrats à partir du 2^{ième} lot d'échantillons de substrats. La figure 10b est un graphe représentant le coefficient R² des résultats de la validation des corrélations établies pour la caractéristique du temps nécessaire pour la production de méthane exprimé en pourcentage de BMP avec le 1ier lot d'échantillons de substrats à partir d'un 2ième lot d'échantillons de substrats.

On observe que les performances de prédiction sont excellentes et valident l'utilisation du modèle de régression PLS. L'erreur d'estimation atteint un maximum autour de 65%. Cela est purement dû à l'expérimentation. En effet la variance expérimentale connaît une allure de courbe similaire à la RMSEP ou SECV, ce qui indique que les temps de dégradation des substrats diffèrent le plus autour de 65% du BMP.

Ainsi, un modèle de prédiction des caractéristiques d'un substrat a été configuré à partir des 31 substrats.

Ce modèle de prédiction a ensuite été utilisé dans un procédé selon l'invention d'estimation des caractéristiques de la biodégradation d'un biodéchet alimentaire par digestion anaérobie dans un méthaniseur.

A partir des données spectrales de ce substrat, on a prédit avec le modèle de prédiction détaillé ci-dessus les caractéristiques de ce substrat.

On a ensuite implémenté un modèle mathématique de digestion anaérobie sur la base de ces caractéristiques du substrat.

Ce modèle mathématique de digestion anaérobie était un modèle ADM1 modifié. Plus précisément, il s'agissait du modèle ADM1 tel que décrit dans la publication Rosén et al. Aspects on ADM1 Implementation within the BSM2 Framework, (2008) qui a été implémenté sous Scilab® et vérifié sur un ensemble de données de référence. Cette vérification est classiquement mise en oeuvre dans ce domaine technique et est parfaitement à la portée de l'homme du métier.

Ensuite, ce modèle ADM1 a été modifié sur la base des travaux décrits dans les publications de Ramirez et al. Modified ADM1 disintegration/hydrolysis structures for modeling batch thermophilic anaerobic digestion of thermally pretreated waste activated sludge, water research (2009), doi: 10.1016/j.watres.2009.05.023 et de Mottet et al. New fractionation for a better bioaccessibility description of particulate organic matter in a modified ADM1 model, Chemical Engineering Journal (2013), doi :10.1016/j.cej.2013.05.082.

Enfin, la dégradation des 4 fractions du substrat suivantes a été implémentée :
- la fraction « Xrc », correspondant à la matière la plus facilement accessible.
- la fraction « Xmc », correspondant à la matière modérément accessible.
- la fraction « Xsc » correspondant à la matière difficilement accessible.
- la fraction « Xne » correspondant à la matière peu ou pas accessible.

Une cinétique de Contois a été utilisée pour décrire la dégradation et l'hydrolyse. Une seule biomasse microbienne a été considérée pour réaliser les étapes d'hydrolyse et de dégradation. Cette biomasse microbienne croît sur l'étape d'hydrolyse. Expérimentalement, l'hydrolyse et la dégradation sont difficiles à différencier. L'étape d'hydrolyse a donc été implémentée comme étape non limitante. L'étape de dégradation étant plus intéressante au niveau du modèle car elle permet d'associer facilement une cinétique de dégradation différente à chaque fraction en gardant une composition biochimique propre à chacune des fractions.

Ce modèle ADM1 a été développé pour répondre à la dégradation de chaque substrat tout en changeant un nombre limité de paramètres.

Les paramètres de compositions en glucides, protéines, lipides, ainsi que la DCO, la biodégradabilité et la cinétique de dégradation doivent être implémentés pour chaque fraction, répondant tout à fait aux paramètres pouvant être prédits à partir d'une analyse de spectroscopie dans le NIR grâce à la présente invention.

La figure 11 est un graphe représentant :
- la courbe expérimentale de production cumulée de méthane en fonction du temps obtenue lors d'une expérience de biodégradation du substrat par digestion anaérobie dans un méthaniseur. Il s'agit de la courbe intitulée « données expérimentales ».
- la courbe prédite de production cumulée de méthane en fonction du temps obtenue à partir de la valeur du BMP du substrat, ainsi que des valeurs des temps nécessaires pour la production de méthane exprimées en pourcentage de BMP du substrat qui ont été prédites à l'étape a). Il s'agit de la courbe intitulée « courbe prédite NIR ».
- la courbe de production cumulée de méthane en fonction du temps obtenue après implémentation dudit modèle mathématique de digestion anaérobie sur la base des caractéristiques prédites avec le modèle de prédiction établi selon le procédé de l'invention à partir des données spectrales du substrat. Il s'agit de la courbe intitulée « données modélisées ».

Au vu de la figure 11, on relève que le procédé d'estimation selon l'invention fournit une excellente estimation de la production cumulée de méthane en fonction du temps qui a été établie à partir des données spectrales du substrat et en utilisant le modèle de prédiction détaillé ci-dessus qui a été configuré à partir de 31 substrats. Ainsi, la figure 11 témoigne de tout l'intérêt de la présente invention qui fournit un procédé d'estimation des caractéristiques de la biodégradation d'un substrat par digestion anaérobie dans un méthaniseur qui est très fiable et performant.

## Revendications

1. Procédé d'estimation d'au moins une des caractéristiques de la biodégradation d'un substrat par digestion anaérobie dans un méthaniseur qui comprend au moins les étapes suivantes :
a) on réceptionne des données spectrales dans le proche infrarouge d'un échantillon dudit substrat en provenance d'un système d'analyse de spectroscopie ou on acquiert des données spectrales dans le proche infrarouge d'un échantillon dudit substrat ;
b) à partir desdites données spectrales dans le proche infrarouge de l'échantillon du substrat, on prédit des caractéristiques dudit substrat avec au moins un modèle de prédiction des caractéristiques d'un substrat destiné à être incorporé dans un méthaniseur pour être biodégradé par digestion anaérobie, lesdites caractéristiques prédites du substrat comprennent au moins :
- le potentiel biochimique de méthane du substrat, abrégé « BMP du substrat »,
- le temps nécessaire pour la production de méthane exprimé en pourcentage de BMP du substrat ;
c) à partir des caractéristiques prédites consistant en le BMP du substrat et en le temps nécessaire pour la production de méthane exprimé en pourcentage de BMP du substrat, on établit une courbe de la quantité cumulée de méthane produit en fonction du temps, ladite courbe est dite « courbe prédite » ;
d) on implémente au moins un modèle mathématique de digestion anaérobie sur la base d'au moins une des caractéristiques du substrat qui ont été prédites à l'étape b) à l'exception de celle du temps nécessaire pour la production de méthane exprimé en pourcentage de BMP du substrat de manière à déterminer une caractéristique de cinétique de dégradation dudit substrat pour laquelle on obtient à partir de ce modèle mathématique de digestion anaérobie une courbe de la quantité cumulée de méthane produit en fonction du temps présentant un écart minimal avec la courbe dite « courbe prédite » établie à l'étape c) ;
e) on implémente au moins un modèle mathématique de digestion anaérobie sur la base de la caractéristique de cinétique de dégradation dudit substrat déterminée à l'étape d) et d'au moins une des caractéristiques du substrat prédites à l'étape b) à l'exception de celle du temps nécessaire pour la production de méthane exprimé en pourcentage de BMP du substrat de façon à déterminer une estimation d'au moins une des caractéristiques de la biodégradation dudit substrat par digestion anaérobie dans un méthaniseur.

2. Procédé d'estimation selon la revendication 1, **caractérisé en ce qu'**à l'étape b), les caractéristiques du substrat qui sont prédites sont en outre au moins l'une des caractéristiques suivantes :
- la composition biochimique du substrat ;
- la demande chimique en oxygène, abrégée « DCO », du substrat ;
- la biodégradabilité du substrat ;
- la fraction de matière organique dégradable ;
- l'accessibilité du substrat ;
- la biodégradabilité du substrat.

3. Procédé d'estimation selon la revendication 2, **caractérisé en ce qu'**au cours de l'étape d) du procédé d'estimation, on implémente le modèle de digestion anaérobie sur la base d'au moins le BMP et la composition biochimique du substrat qui ont été prédits à l'étape b) et **en ce qu'**au cours de l'étape e) du procédé d'estimation, on implémente le modèle de digestion anaérobie sur la base de la caractéristique de la cinétique de dégradation du substrat qui a été déterminée à l'étape d), ainsi que d'au moins le BMP et la composition biochimique du substrat qui ont été prédits à l'étape b).

4. Procédé d'estimation selon l'une quelconque des revendications 1 à 3, **caractérisé en ce que** la caractéristique du substrat prédite à l'étape b) du temps nécessaire pour la production de méthane exprimé en pourcentage de BMP du substrat consiste en un ensemble de n valeurs des temps nécessaires pour la production de respectivement n pourcentages de BMP du substrat, n étant un nombre entier supérieur ou égal à 1, de préférence supérieur ou égal à 2, plus préférentiellement compris entre 2 et 100.

5. Procédé d'estimation selon l'une quelconque des revendications 1 à 4, **caractérisé en ce que** le modèle mathématique de digestion anaérobie de l'étape e) est en outre implémenté sur la base d'au moins un paramètre de fonctionnement du méthaniseur.

6. Procédé d'estimation selon l'une quelconque des revendications 1 à 5, **caractérisé en ce qu'**aux étapes d) et e), le modèle mathématique de digestion anaérobie est un modèle ADM1 ou l'une des variantes du modèle ADM1.

7. Procédé d'estimation selon l'une quelconque des revendication 1 à 6, **caractérisé en ce qu'**on traite avec au moins une méthode chimiométrique les données spectrales de l'étape a) de manière à obtenir des données spectrales traitées de l'échantillon de substrat.

8. Procédé d'estimation selon l'une quelconque des revendication 1 à 7, **caractérisé en ce qu'**il est mis en oeuvre par une unité de traitement informatique.

9. Procédé d'estimation selon l'une quelconque des revendications 1 à 8, **caractérisé en ce que** le substrat est choisi parmi :
- les eaux usées industrielles, les eaux usées urbaines ;
- les boues de station d'épuration ;
- les micro-algues ;
- les déchets municipaux dont notamment les déchets alimentaires ;
- les déchets industriels ;
- les déchets de l'agriculture ;
pris seuls ou en mélanges.

10. Procédé d'estimation selon l'une quelconque des revendications 1 à 9, **caractérisé en ce que** les caractéristiques de la biodégradation d'un substrat par digestion anaérobie dans un méthaniseur sont choisies parmi au moins l'une des caractéristiques suivantes :
- la production cumulée de méthane en fonction du temps par le méthaniseur;
- la composition du biogaz produit ;
- la concentration en produits intermédiaires et en inhibiteurs ;
- le pourcentage de DCO de substrat biodégradé.

11. Procédé d'estimation selon l'une quelconque des revendications 1 à 10, **caractérisé en ce que** le modèle de prédiction des caractéristiques d'un substrat a été établi avec un procédé de configuration d'un modèle de prédiction des caractéristiques d'un substrat destiné à être incorporé dans un méthaniseur pour être biodégradé par digestion anaérobie qui comprend au moins les étapes suivantes :
1) on dispose d'un ensemble d'échantillons de substrats d'un méthaniseur;
2) on soumet ledit ensemble d'échantillons de substrats à une analyse de spectroscopie dans le proche infrarouge de manière à acquérir des données spectrales de chaque échantillon de substrat ;
3) on réalise des analyses de laboratoire déterminées sur chaque échantillon de substrat de manière à obtenir pour chaque échantillon de substrat au moins les caractéristiques expérimentales suivantes :
- le BMP dudit échantillon de substrat,
- le temps nécessaire pour la production de méthane exprimé en pourcentage de BMP de l'échantillon de substrat ;
4) on corrèle les données spectrales de l'étape 2) avec les caractéristiques expérimentales obtenues à l'étape 3) de manière à établir un modèle de prédiction qui est configuré pour prédire des caractéristiques d'un substrat destiné à être incorporé dans un méthaniseur à partir de données spectrales d'un échantillon de ce substrat.

12. Procédé d'estimation selon la revendication 11, **caractérisé en ce qu'**à l'étape 4) on corrèle les données spectrales de l'étape 2) avec les caractéristiques expérimentales obtenues à l'étape 3) avec au moins un traitement mathématique.

13. Procédé d'estimation selon la revendication 12, **caractérisé en ce que** le traitement mathématique consiste en une régression des moindres carrés partiels.

## Patentansprüche

1. Verfahren zur Schätzung von mindestens einer der Charakteristiken des biologischen Abbaus eines Substrats durch anaerobe Vergärung in einem Methanreaktor, das mindestens die folgenden Schritte umfasst:
a) von einer Probe des Substrats werden Spektraldaten im nahen Infrarotbereich empfangen, die aus einem spektroskopischen Analysesystem stammen, oder von einer Probe des Substrats werden Spektraldaten im nahen Infrarotbereich erfasst;
b) anhand der Spektraldaten im nahen Infrarotbereich der Probe des Substrats werden Charakteristiken des Substrats mit mindestens einem Modell zur Vorhersage der Charakteristiken eines Substrats vorhergesagt, das dazu bestimmt ist, in einen Methanreaktor eingebracht zu werden, um durch anaerobe Vergärung biologisch abgebaut zu werden, wobei die vorhergesagten Charakteristiken des Substrats mindestens umfassen:
- das biochemische Methanpotential des Substrats, abgekürzt "BMP des Substrats",
- die Zeit, die für die Produktion von Methan erforderlich ist, ausgedrückt in BMP-Prozent des Substrats;
c) anhand der vorhergesagten Charakteristiken, die im BMP des Substrats und in der Zeit bestehen, die für die Produktion von Methan erforderlich ist, ausgedrückt in BMP-Prozent des Substrats, wird eine Kurve der kumulierten produzierten Methanmenge in Abhängigkeit von der Zeit erstellt, wobei die Kurve als "vorhergesagte Kurve" bezeichnet wird;
d) auf der Basis von mindestens einer der Charakteristiken des Substrats, die im Schritt b) vorhergesagt wurden, mit Ausnahme derjenigen der Zeit, die für die Produktion von Methan erforderlich ist, ausgedrückt in BMP-Prozent des Substrats, wird mindestens ein mathematisches anaerobes Vergärungsmodell implementiert, um eine Abbaukinetik-Charakteristik des Substrats zu bestimmen, für die anhand dieses mathematischen anaeroben Vergärungsmodells eine Kurve der kumulierten produzierten Methanmenge in Abhängigkeit von der Zeit erhalten wird, die einen Mindestabstand zu der im Schritt c) erstellten, als "vorhergesagte Kurve" bezeichneten Kurve aufweist;
e) auf der Basis der Abbaukinetik-Charakteristik des Substrats, die im Schritt d) bestimmt wurde, und von mindestens einer der Charakteristiken des Substrats, die im Schritt b) vorhergesagt wurden, mit Ausnahme derjenigen der Zeit, die für die Produktion von Methan erforderlich ist, ausgedrückt in BMP-Prozent des Substrats, wird mindestens ein mathematisches anaerobes Vergärungsmodell implementiert, um eine Schätzung von mindestens einer der Charakteristiken des biologischen Abbaus des Substrats durch anaerobe Vergärung in einem Methanreaktor zu bestimmen.

2. Schätzverfahren nach Anspruch 1, **dadurch gekennzeichnet, dass** im Schritt b) die Charakteristiken des Substrats, die vorhergesagt werden, weiter mindestens eine der folgenden Charakteristiken sind:
- die biochemische Zusammensetzung des Substrats;
- der chemische Sauerstoffbedarf, abgekürzt "CSB", des Substrats;
- die biologische Abbaubarkeit des Substrats;
- der Anteil an abbaubarem organischem Material;
- die Verfügbarkeit des Substrats;
- die biologische Abbaubarkeit des Substrats.

3. Schätzverfahren nach Anspruch 2, **dadurch gekennzeichnet, dass** im Laufe des Schritts d) des Schätzverfahrens das anaerobe Vergärungsmodell auf der Basis von mindestens dem BMP und der biochemischen Zusammensetzung des Substrats implementiert wird, die im Schritt b) vorhergesagt wurden, und dadurch, dass im Laufe des Schritts e) des Schätzverfahrens das anaerobe Vergärungsmodell auf der Basis der Charakteristik der Abbaukinetik des Substrats, die im Schritt d) bestimmt wurde, sowie mindestens des BMP und der chemischen Zusammensetzung des Substrats, die im Schritt b) vorhergesagt wurden, implementiert wird.

4. Schätzverfahren nach einem der Ansprüche 1 bis 3, **dadurch gekennzeichnet, dass** die im Schritt b) vorhergesagte Charakteristik des Substrats der Zeit, die für die Produktion von Methan erforderlich ist, ausgedrückt in BMP-Prozent des Substrats, in einem Satz von n Werten der Zeiten besteht, die für die Produktion von jeweils n BMP-Prozent des Substrats erforderlich sind, wobei n eine ganze Zahl größer oder gleich 1, vorzugsweise größer oder gleich 2, stärker bevorzugt im Bereich zwischen 2 und 100 ist.

5. Schätzverfahren nach einem der Ansprüche 1 bis 4, **dadurch gekennzeichnet, dass** das mathematische anaerobe Vergärungsmodell aus dem Schritt e) weiter auf der Basis von mindestens einem Betriebsparameter des Methanreaktors implementiert wird.

6. Schätzverfahren nach einem der Ansprüche 1 bis 5, **dadurch gekennzeichnet, dass** in den Schritten d) und e) das mathematische anaerobe Vergärungsmodell ein ADM1-Modell oder eine der Varianten des ADM1-Modells ist.

7. Schätzverfahren nach einem der Ansprüche 1 bis 6, **dadurch gekennzeichnet, dass** die Spektraldaten aus dem Schritt a) mit mindestens einer chemometrischen Methode verarbeitet werden, um verarbeitete Spektraldaten der Substratprobe zu erhalten.

8. Schätzverfahren nach einem der Ansprüche 1 bis 7, **dadurch gekennzeichnet, dass** es von einer Informationsverarbeitungseinheit umgesetzt wird.

9. Schätzverfahren nach einem der Ansprüche 1 bis 8, **dadurch gekennzeichnet, dass** das Substrat ausgewählt ist aus:
- den industriellen Abwässern, den städtischen Abwässern;
- den Klärschlämmen;
- den Mikroalgen;
- den kommunalen Abfällen, darunter insbesondere den Lebensmittelabfällen;
- den industriellen Abfällen;
- den Abfällen aus der Landwirtschaft;
einzeln genommen oder in Mischungen.

10. Schätzverfahren nach einem der Ansprüche 1 bis 9, **dadurch gekennzeichnet, dass** die Charakteristiken des biologischen Abbaus eines Substrats durch anaerobe Vergärung in einem Methanreaktor aus mindestens einer der folgenden Charakteristiken ausgewählt sind:
- der kumulierten Methanproduktion durch den Methanreaktor in Abhängigkeit von der Zeit;
- der Zusammensetzung des produzierten Biogases;
- der Konzentration an Zwischenprodukten und an Hemmstoffen;
- den CSB-Prozent des biologisch abgebauten Substrats.

11. Schätzverfahren nach einem der Ansprüche 1 bis 10, **dadurch gekennzeichnet, dass** das Modell zur Vorhersage der Charakteristiken eines Substrats erstellt wurde mit einem Verfahren zur Konfiguration eines Modells zur Vorhersage der Charakteristiken eines Substrats, das dazu bestimmt ist, in einen Methanreaktor eingebracht zu werden, um durch anaerobe Vergärung biologisch abgebaut zu werden, welches mindestens die folgenden Schritte umfasst:
1) es wird über einen Satz von Substratproben eines Methanreaktors verfügt;
2) der Satz von Substratproben wird einer spektroskopischen Analyse im nahen Infrarotbereich unterzogen, um Spektraldaten von jeder Substratprobe zu erfassen;
3) an jeder Substratprobe werden bestimmte Laboranalysen ausgeführt, um für jede Substratprobe mindestens die folgenden experimentellen Charakteristiken zu erhalten:
- das BMP der Substratprobe,
- die Zeit, die für die Produktion von Methan erforderlich ist, ausgedrückt in BMP-Prozent der Substratprobe;
4) die Spektraldaten aus dem Schritt 2) werden mit den im Schritt 3) erhaltenen experimentellen Charakteristiken korreliert, um ein Vorhersagemodell zu erstellen, das dafür konfiguriert ist, Charakteristiken eines Substrats, das dazu bestimmt ist, in einen Methanreaktor eingebracht zu werden, anhand von Spektraldaten einer Probe dieses Substrats vorherzusagen.

12. Schätzverfahren nach Anspruch 11, **dadurch gekennzeichnet, dass** im Schritt 4) die Spektraldaten aus dem Schritt 2) mit mindestens einer mathematischen Verarbeitung mit den im Schritt 3) erhaltenen experimentellen Charakteristiken korreliert werden.

13. Schätzverfahren nach Anspruch 12, **dadurch gekennzeichnet, dass** die mathematische Verarbeitung in einer Partial Least Squares-Regression besteht.

## Claims

1. A method for estimating at least one of the characteristics of the biodegradation of a substrate by anaerobic digestion in a methanizer which comprises at least the following steps:
a) spectral data in the near infrared of a sample of said substrate from a spectroscopy analysis system are received or spectral data in the near infrared of a sample of said substrate are acquired;
b) characteristics of said substrate are predicted from said spectral data in the near infrared of the sample of the substrate, with at least one model for predicting characteristics of a substrate intended to be incorporated into a methanizer in order to be biodegraded by anaerobic digestion, said predicted characteristics of the substrate comprise at least:
- the biochemical methane potential of the substrate, abbreviated «BMP of the substrate»,
- time required for the production of methane expressed in percentage of BMP of the substrate;
c) from the predicted characteristics consisting in the BMP of the substrate and in the time required for methane production expressed in percentage of BMP of the substrate, a curve of the cumulative amount of methane produced as a function of time is established, said curve is called «predicted curve»;
d) at least one mathematical model of anaerobic digestion is implemented based on at least one of the characteristics of the substrate that have been predicted in step b) except that of the time required for methane production expressed in percentage of BMP of the substrate so as to determine a characteristic of degradation kinetics of said substrate for which a curve of the cumulative amount of methane produced as a function of time having a minimum deviation from the curve called «predicted curve» (established in step c) is obtained from this mathematical model of anaerobic digestion;
e) at least one mathematical model of anaerobic digestion is implemented based on the characteristic of degradation kinetics of said substrate determined in step d) and on at least one of the characteristics of the substrate predicted in step b) except that of the time required for methane production expressed in percentage of BMP of the substrate so as to determine an estimation of at least one of the characteristics of the biodegradation of said substrate by anaerobic digestion in a methanizer.

2. The estimation method according to claim 1, **characterized in that** in step b), the characteristics of the substrate that are predicted are furthermore at least one of the following characteristics:
- the biochemical composition of the substrate;
- the chemical oxygen demand, abbreviated "COD", of the substrate;
- the biodegradability of the substrate;
- the fraction of degradable organic matter;
- the accessibility of the substrate;
- the biodegradability of the substrate.

3. The estimation method according to claim 2, **characterized in that**, during step d) of the estimation method, the anaerobic digestion model is implemented based on at least the BMP and the biochemical composition of the substrate that have been predicted in step b) and **in that**, during step e) of the estimation method, the anaerobic digestion model is implemented based on the characteristic of the degradation kinetics of the substrate that has been determined in step d), as well as on at least the BMP and the biochemical composition of the substrate that have been predicted in step b).

4. The estimation method according to any one of claims 1 to 3, **characterized in that** the characteristic of the substrate predicted in step b) of the time required for methane production expressed in percentage of BMP of the substrate consists of a set of n values of the times required for the production of respectively n percentages of BMP of the substrate, n being an integer greater than or equal to 1, preferably greater than or equal to 2, more preferably comprised between 2 and 100.

5. The estimation method according to any one of claims 1 to 4, **characterized in that** the mathematical model of anaerobic digestion of step e) is furthermore implemented based on at least one operating parameter of the methanizer.

6. The estimation method according to any one of claims 1 to 5, **characterized in that**, in steps d) and e), the mathematical model of anaerobic digestion is an ADM1 model or one of the variants of the model ADM1.

7. The estimation method according to any one of claims 1 to 6, **characterized in that** the spectral data of step a) are processed with at least one chemometric method so as to obtain processed spectral data of the substrate sample.

8. The estimation method according to any one of claims 1 to 7, **characterized in that** it is implemented by a computer processing unit.

9. The estimation method according to any one of claims 1 to 8, **characterized in that** the substrate is selected from:
- industrial wastewater, urban wastewater;
- sewage sludge;
- microalgae;
- municipal waste including in particular food waste;
- industrial waste;
- agricultural waste;
taken alone or in mixtures.

10. The estimation method according to any one of claims 1 to 9, **characterized in that** the characteristics of the biodegradation of a substrate by anaerobic digestion in a methanizer are selected from at least one of the following characteristics:
- the cumulative methane production by the methanizer as a function of time;
- the composition of the produced biogas;
- the concentration of intermediate products and of inhibitors;
- the percentage of COD of biodegraded substrate.

11. The estimation method according to any one of claims 1 to 10, **characterized in that** the model for predicting the characteristics of a substrate has been established with a method for configuring a model for predicting the characteristics of a substrate intended to be incorporated into a methanizer in order to be biodegraded by anaerobic digestion which comprises at least the following steps:
1) a set of substrate samples from a methanizer are provided;
2) said set of substrate samples are subjected to a near infrared spectroscopy analysis so as to acquire spectral data from each substrate sample;
3) determined laboratory analysis are performed on each substrate sample so as to obtain for each substrate sample at least the following experimental characteristics:
- the BMP of said substrate sample,
- the time required for methane production expressed in percentage of BMP of the substrate sample;
4) the spectral data of step 2) are correlated with the experimental characteristics obtained in step 3) so as to establish a prediction model that is configured to predict characteristics of a substrate intended to be incorporated into a methanizer from spectral data of a sample of this substrate.

12. The estimation method according to claim 11, **characterized in that**, in step 4), the spectral data of step 2) are correlated with the experimental characteristics obtained in step 3) with at least one mathematical processing.

13. The estimation method according to claim 12, **characterized in that** the mathematical processing consists of a partial least squares regression.
